# EUROPEAN PATENT APPLICATION

(11) **EP 3 552 622 A2**
(43) Date of publication of application: **16.10.2019**
(21) Application number: 19173464.9
(22) Date of filing: 20.11.2009
(51) Int. Cl.: A61K 39/00, A61K 39/39, A61K 48/00, C12N 15/62, C07K 19/00

(54) **PRIMING OF AN IMMUNE RESPONSE**

(30) Priority: 21.11.2008 DK 200801638
(62) Divisional of application: 15152408.9
(71) Applicant: Københavns Universitet (University of Copenhagen), 1017 Copenhagen K (DK)
(72) Inventor: HOLST, Peter, 2700 Bronshoj (DK); THOMSEN, Allan, 2100 Copenhagen (DK); CHRISTENSEN, Jan, 2650 Hvidovre (DK); GRUJIC, Mirjana, 129 48 Hagersten (SE)
(74) Representative: Ruscoe, Peter James

(57) **Abstract**

There is provided inter alia a nucleic acid construct comprising sequences encoding a) at least one invariant chain or variant thereof operatively linked to b) at least one antigenic protein or peptide or an antigenic fragment of said protein or peptide, wherein said nucleic acid construct is used for priming of a cancer vaccine, and/or said invariant chain or variant thereof does not comprise the first 17 amino acids, and/or said invariant chain or variant thereof does not comprise the LRMK amino acid residues of the KEY region, and/or said invariant chain comprises a variant of the CLIP region.

SEQUENCE LISTING

<110> Holst, Peter Johannes
      Thomsen, Allan Randrup
      Christensen, Jan Pravsgaard
      Grujic, Mirjana

<120> PRIMING OF AN IMMUNE RESPONSE

<130> P1982DK00

<140> PA 2008 01638
<141> 2008-11-21

<160> 4

<170> FastSEQ for Windows Version 4.0

<210> 1
<211> 1287
<212> DNA
<213> Homo Sapiens - Invariant chain

<400> 1
ctgcaggggg gggggggggg gggggggaca ttggctcttc cttggggagt gatgcacagg 60
aggagaagca ggagctgtcg ggaagatcag aagccagtca tggatgacca gcgcgacctt 120
atctccaaca atgagcaact gcccatgctg ggccggcgcc ctggggcccc ggagagcaag 180
tgcagccgcg gagccctgta cacaggcttt tccatcctgg tgactctgct cctcgctggc 240
caggccacca ccgcctactt cctgtaccag cagcagggcc ggctggacaa actgacagtc 300
acctcccaga acctgcagct ggagaacctg cgcatgaagc ttcccaagcc tcccaagcct 360
gtgagcaaga tgcgcatggc caccccgctg ctgatgcagg cgctgcccat gggagccctg 420
ccccaggggc ccatgcagaa tgccaccaag tatggcaaca tgacagagga ccatgtgatg 480
cacctgctcc agaatgctga ccccctgaag gtgtacccgc cactgaaggg gagcttcccg 540
gagaacctga gacaccttaa gaacaccatg gagaccatag actggaaggt ctttgagagc 600
tggatgcacc attggctcct gtttgaaatg agcaggcact ccttggagca aaagcccact 660
gacgctccac cgaaagagtc actggaactg gaggacccgt cttctgggct gggtgtgacc 720
aagcaggatc tgggcccagt ccccatgtga gagcagcaga ggcggtcttc aacatcctgc 780
cagccccaca cagctacagc tttcttgctc ccttcagccc ccagcccctc ccccatctcc 840
caccctgtac ctcatcccat gagaccctgg tgcctggctc tttcgtcacc cttggacaag 900
acaaaccaag tcggaacagc agataacaat gcagcaaggc cctgctgccc aatctccatc 960
tgtcaacagg ggcgtgaggt cccaggaagt ggccaaaagc tagacagatc cccgttcctg 1020
acatcacagc agcctccaac acaaggctcc aagacctagg ctcatggacg agatgggaag 1080
gcacagggag aagggataac cctacaccca gaccccaggc tggacatgct gactgtcctc 1140
tcccctccag cctttggcct tggcttttct agcctattta cctgcaggct gagccactct 1200
cttccctttc cccagcatca ctccccaagg aagagccaat gttttccacc catccctccc 1260
cccccccccc cccccccccc cctgcag 1287

<210> 2
<211> 216
<212> PRT
<213> Homo Sapiens - Invariant chain

<400> 2
Met Asp Asp Gln Arg Asp Leu Ile Ser Asn Asn Glu Gln Leu Pro Met
 1               5                  10                  15
Leu Gly Arg Arg Pro Gly Ala Pro Glu Ser Lys Cys Ser Arg Gly Ala
            20                  25                  30
Leu Tyr Thr Gly Phe Ser Ile Leu Val Thr Leu Leu Leu Ala Gly Gln
        35                  40                  45
Ala Thr Thr Ala Tyr Phe Leu Tyr Gln Gln Gln Gly Arg Leu Asp Lys
    50                  55                  60

Leu Thr Val Thr Ser Gln Asn Leu Gln Leu Glu Asn Leu Arg Met Lys
65                  70                  75                  80
Leu Pro Lys Pro Pro Lys Pro Val Ser Lys Met Arg Met Ala Thr Pro
                85                  90                  95
Leu Leu Met Gln Ala Leu Pro Met Gly Ala Leu Pro Gln Gly Pro Met
            100                 105                 110
Gln Asn Ala Thr Lys Tyr Gly Asn Met Thr Glu Asp His Val Met His
        115                 120                 125
Leu Leu Gln Asn Ala Asp Pro Leu Lys Val Tyr Pro Pro Leu Lys Gly
    130                 135                 140
Ser Phe Pro Glu Asn Leu Arg His Leu Lys Asn Thr Met Glu Thr Ile
145                 150                 155                 160
Asp Trp Lys Val Phe Glu Ser Trp Met His His Trp Leu Leu Phe Glu
                165                 170                 175
Met Ser Arg His Ser Leu Glu Gln Lys Pro Thr Asp Ala Pro Pro Lys
            180                 185                 190
Glu Ser Leu Glu Leu Glu Asp Pro Ser Ser Gly Leu Gly Val Thr Lys
        195                 200                 205
Gln Asp Leu Gly Pro Val Pro Met
    210                 215

<210> 3
<211> 648
<212> DNA
<213> Mus Musculus - Invariant chain

<400> 3
atggatgacc aacgcgacct catctctaac catgaacagt tgcccatact gggcaaccgc 60
cctagagagc cagaaaggtg cagccgtgga gctctgtaca ccggtgtctc tgtcctggtg 120
gctctgctct tggctgggca ggccaccact gcttacttcc tgtaccagca acagggccgc 180
ctagacaagc tgaccatcac ctcccagaac ctgcaactgg agagccttcg catgaagctt 240
ccgaaatctg ccaaacctgt gagccagatg cggatggcta ctcccttgct gatgcgtcca 300
atgtccatgg ataacatgct ccttgggcct gtgaagaacg ttaccaagta cggcaacatg 360
acccaggacc atgtgatgca tctgctcacg aggtctggac ccctggagta cccgcagctg 420
aaggggacct tcccagagaa tctgaagcat cttaagaact ccatggatgg cgtgaactgg 480
aagatcttcg agagctggat gaagcagtgg ctcttgtttg agatgagcaa gaactccctg 540
gaggagaaga agcccaccga ggctccacct aaagagccac tggacatgga agacctatct 600
tctggcctgg gagtgaccag gcaggaactg ggtcaagtca ccctgtga             648

<210> 4
<211> 215
<212> PRT
<213> Mus Musculus - Invariant chain

<400> 4
Met Asp Asp Gln Arg Asp Leu Ile Ser Asn His Glu Gln Leu Pro Ile
 1               5                  10                  15
Leu Gly Asn Arg Pro Arg Glu Pro Glu Arg Cys Ser Arg Gly Ala Leu
            20                  25                  30
Tyr Thr Gly Val Ser Val Leu Val Ala Leu Leu Leu Ala Gly Gln Ala
        35                  40                  45
Thr Thr Ala Tyr Phe Leu Tyr Gln Gln Gln Gly Arg Leu Asp Lys Leu
    50                  55                  60
Thr Ile Thr Ser Gln Asn Leu Gln Leu Glu Ser Leu Arg Met Lys Leu
65                  70                  75                  80
Pro Lys Ser Ala Lys Pro Val Ser Gln Met Arg Met Ala Thr Pro Leu
                85                  90                  95
Leu Met Arg Pro Met Ser Met Asp Asn Met Leu Leu Gly Pro Val Lys
            100                 105                 110
Asn Val Thr Lys Tyr Gly Asn Met Thr Gln Asp His Val Met His Leu
        115                 120                 125
Leu Thr Arg Ser Gly Pro Leu Glu Tyr Pro Gln Leu Lys Gly Thr Phe

    130                 135                 140
Pro Glu Asn Leu Lys His Leu Lys Asn Ser Met Asp Gly Val Asn Trp
145                 150                 155                 160
Lys Ile Phe Glu Ser Trp Met Lys Gln Trp Leu Leu Phe Glu Met Ser
                165                 170                 175
Lys Asn Ser Leu Glu Glu Lys Lys Pro Thr Glu Ala Pro Pro Lys Glu
            180                 185                 190
Pro Leu Asp Met Glu Asp Leu Ser Ser Gly Leu Gly Val Thr Arg Gln
        195                 200                 205
Glu Leu Gly Gln Val Thr Leu
    210                 215

## Description

All patent and non-patent references cited in the present application, are hereby incorporated by reference in their entirety.

### Field of invention

The present invention relates to a technology and method of priming of an immune response using invariant chain linked antigen, when these are used to prime a subsequent booster immunization using any suitable vaccine.

### Background of invention

Vaccination is the administration of an antigenic material (a vaccine) to a subject in order to produce immunity to a disease or condition. When used to stimulate an immune response, the antigen is known as an immunogen, and the process is known as immunization. Vaccinations involve the administration of one or more immunogens, which can be administered in several forms.

Vaccination requires the establishment of a solid immune response. The immune response that is activated by infection or vaccination depends on the interaction of several cell types, such as T-, B- and antigen presenting cells as well as several different molecules, primarily antigens, MHC molecules, T- and B-cells receptors and many more.

Traditional vaccines, or first generation vaccines, are based on killed or attenuated pathogenic strains. These often suffer from reduced infectivity and they are often insufficiently immunogenic, resulting in inadequate protection from the vaccination.

Development of second generation vaccines, or subunit vaccines, based on individual antigenic proteins from the pathogenic organisms has revealed that pure peptides or carbohydrates tend to be weak immunogens.

DNA vaccines, or third generation vaccines, have the ability to induce a wider range of immune response types, but maintains the potential disadvantage of having low immunogenicity in humans.

For all types of vaccines, vaccination programs are faced with an unmet need for increasing vaccine potency, to overcome the limitations cited above and provide a more cost-effective means of stimulating the immune system during vaccination.

The present invention is targeted at solving the problem associated with low immunogenicity of vaccines by proving a solution for increasing the potency of vaccines.

The present invention is targeted at solving the problem associated with low immunogenicity of vaccines by proving a solution for priming of an immune response.

The present invention is thus directed to priming the immune system with a nucleic acid construct comprising MHC class II associated invariant chain/CD74 (herein referred to as invariant chain or li) or a variant thereof and encoding at least one antigenic protein or a fragment of said antigenic protein, followed by a subsequent booster vaccination to increase the potency of said vaccine.

Vaccines according to the present invention may be directed to a pathogenic antigen or a cancer antigen.

Data presented herein shows that it is not straightforward to develop prime-boost regimens using nucleic acid constructs comprising invariant chain or variant thereof.

Surprisingly, the present invention discloses that the li-KEY (comprising LRMK amino acid residues) and/or part of the li-CLIP domain from the invariant chain may be altered without reducing the effects of said immune priming.

Prior art references that have inadequately addressed this issue are discussed below:
WO 2007/062656 (Holst et al.) is directed to developing improved DNA vaccines to stimulate the immune response in a manner that increases the kinetics of the response, simultaneously with both broadening and improving the response. Holst et al. found that fusion of an antigen to the invariant chain dramatically enhanced the ensuing antiviral CD4⁺ and CD8⁺ T-cell responses through a CD4⁺ T-cell independent mechanism.

Holmes et al. (J Clin Oncol. 2008, Jul 10;26(20):3426-33) describe the first human phase I trial of an li-key hybrid peptide vaccine, wherein the li-key comprises the LRMK four-amino-acid sequence, a central portion of the invariant chain protein.

The finding of Kallinteris et al. (Expert Opin. Biol. Ther. 2006, 6(12):1311,1321) is also directed at utilising the li-key moiety comprising the LRMK amino acids for enhancing vaccine potency.

US 2008/0095798 (Humphreys et al.) disclose a method for increasing the potency of a vaccine against a pathogen by first priming a subject's immune system with an li-Key hybrid peptide construct comprising the LRMK residues of said li-key peptide, and subsequently administering a vaccine against a pathogen to boost the immune response raised in the priming step.

### Summary of invention

The present invention shows that priming the immune system with a nucleic acid construct comprising at least one MHC class II associated invariant chain/CD74 (herein referred to as invariant chain or li) or a variant thereof and encoding at least one antigenic protein or a fragment of said antigenic protein, followed by a subsequent booster vaccination increases the potency of said vaccine.

Vaccines according to the present invention may be directed to a pathogenic antigen or a cancer antigen.

Surprisingly, the present invention discloses that the li-KEY domain (comprising LRMK amino acid residues) and/or part of the li-CLIP domain may be partly substituted or omitted from the invariant chain without reducing the effects of said priming.

Thus, the present invention has solved the problem of adequately stimulating the immune response raised by vaccination by employing a dual step prime-boost regimen, whereby the immune system is first primed with a nucleic acid construct comprising invariant chain or a variant thereof followed by subsequent booster vaccination using any type of suitable vaccine, in a manner that increases the kinetics of the response, simultaneously with both broadening and improving the response. In particular, a novel system for a directed, specific and fast stimulation of the immune system is hereby made available in order to improve the vaccination regimens of all animals, such as humans.

This problem is solved by the embodiments of the present invention characterized in the claims. By the present invention it was found that priming with an li chain based nucleic acid construct significantly augments the generation of antigen-specific CD8⁺ T-cells, CD4⁺ T-cells and/or B-cells upon subsequent boosting with a vaccine.

It is thus an aspect of the present invention to provide a nucleic acid construct comprising sequences encoding at least one invariant chain or a variant thereof operatively linked to at least one antigenic protein or peptide or an antigenic fragment of said protein or peptide, wherein said nucleic acid construct is capable of priming the immune system to enhance immunization upon administration of a subsequent vaccine in a subject.

The present invention in one embodiment provides a nucleic acid construct comprising sequences encoding at least one invariant chain or variant thereof operatively linked to at least one antigenic protein or peptide or an antigenic fragment of said protein or peptide, wherein said nucleic acid construct is capable of priming immunization by administration of a subsequent vaccine in a subject.

In one embodiment, the invariant chain comprised in the nucleic acid construct of the present invention may be altered from its wild type sequence without reducing the effect of li.

Thus, in one embodiment, the li-KEY domain comprising the LRMK amino acid residues have been altered by deletion or substitution such as mutation.

In another embodiment, part of the li-CLIP-domain has been altered by deletion or substitution such as mutation. The li-CLIP domain may specifically be altered by substituting Methionine on position 91 and 99 to Alanine; this surprisingly increases the MHCII presentation.

In another embodiment li may specifically be altered by deleting the first 17 amino acids of li; this surprisingly increases the memory response.

It follows that each of these alterations may occur separately or in combination.

In one embodiment, the invariant chain comprised in the nucleic acid construct of the present invention is altered from its wild type sequence when used for priming the immune response of a vaccine directed at a virus, a microorganism such as a bacteria or a parasite.

In another embodiment, the invariant chain comprised in the nucleic acid construct of the present invention may or may not be altered from its wild type sequence when used for priming the immune response of a cancer vaccine or a vaccine directed at an abnormal physiological response.

In another embodiment, at least one part of the nucleic acid construct used to prime the immune response and the subsequent vaccine used to boost the immune response are identical. Said at least one identical part of the primer and the booster may be li or a variant thereof, the antigenic peptide or part of the antigenic peptide, or a ubiquitous helper T-cell epitope.

It is likewise an object of the present invention to provide a delivery vehicle comprising the nucleic acid construct as detailed herein, wherein said delivery vehicle is an RNA based vehicle, a DNA based vehicle/ vector, a lipid based vehicle, a polymer based vehicle or a virally derived DNA or RNA vehicle.

In a preferred embodiment, said delivery vehicle comprises the formation of liposomes, formation of biodegradable polymer microspheres, coating of the nucleic acid construct onto colloidal gold particles or incorporation into a virally derived DNA or RNA vector.

In yet a preferred embodiment, said nucleic acid construct or said delivery vehicle is administered by means of needle injection, gene gun, jet injection, electroporation, ultrasound, or hydrodynamic delivery.

Thus it is an aspect of the present invention to provide a means of stimulating an immune response by a nucleic acid construct comprising sequences encoding at least one invariant chain or variant thereof operatively linked to at least one antigenic protein or peptide or an antigenic fragment of said protein or peptide.

A further object provides means of stimulating intercellular spreading of the nucleic acid construct or the proteins encoded within any of these or any parts of any of these.

It is yet an object of the present invention to provide a chimeric protein as encoded by the nucleic acid construct described herein.

It is further an aspect of the present invention to provide an antibody that recognizes the chimeric protein encoded by the nucleic acid construct described herein.

It is an aspect of the present invention to provide a method for improving the potency of a vaccine comprising administering the nucleic acid construct as detailed herein.

Especially relevant to the present invention is a nucleic acid construct comprising sequences encoding at least one invariant chain or variant thereof operatively linked to at least one antigenic protein or peptide or an antigenic fragment of said protein or peptide which is suitable for priming of an immune response.

It is yet an aspect of the present invention to provide a kit in parts, said kit comprising a nucleic acid construct as described herein together with a medical instrument or other means of administering said nucleic acid construct, and/or a suitable vaccine, and furthermore instructions on how to use the kit in parts.

It follows that the present invention provides means for potentiating an immune response in an animal, by administering to the animal a nucleic acid construct as detailed herein below.

### Description of Drawings

**Figure 1****:** DNA-priming with an li chain based naked DNA vaccine.
**Figure 2****:** Location of the domains and the tested mutations in the li sequence.
**Figure 3****:** li dramatically increases cell surface presentation of the SIINFEKUH-2kb OVA derived epitope.
**Figure 4****:** li works only in cis.
**Figure 5****:** N-terminal deletions and substitutions does not effect li stimulatory capacity.
**Figure 6****:** C-terminal deletions and substitutions does not effect li stimulatory capacity.
**Figure 7****:** Only a N- and C-terminal deletion reduces li stimulatory capacity.
**Figure 8****:** Dose-response of Ad-liGP and Ad-GP vaccines.
**Figure 9****:** Comparison of Ad-GP, Ad-liGP and Ad-liCLIPGP for MHC class II presentation.
**Figure 10****:** Comparison of Ad-GP, Ad-liGP, Ad-GPLamp-1 and Ad-liΔ17GP in an in vivo time-course study.
**Figure 11****:** Comparison of Ad-GP, Ad-liGP, Ad-liΔI7GP, Ad-liKEYGP, Ad-liCLIPGP, Ad-li1-117GP and Ad-li1-199GP in vivo responses.
**Figure 12****:** Ad-GP is capable of priming a subsequent Ad-liGP boost.
**Figure 13****:** Ad-liGP is not capable of priming a subsequent Ad-GP or Ad-liGP boost.
**Figure 14****:** Dose-response of Ad-GP and Adli-Gp vaccines.
**Figure 15****:** The Mannose receptor coupled to a variant of invariant chain comprising residues 50 to 215 (Ii50-215), further coupled to an adenoviral fiber protein.

### Definitions

**Adenovirus:** A group of double-stranded DNA containing viruses. Adenoviruses can be genetically modified making them replication incompetent or conditionally replication incompetent. In this form, as adenoviral constructs or adenovectors, they can be used as gene delivery vehicles for vaccination or gene therapy.

**Adenoviral fiber protein:** a fiber protein from any seratype of adenovirus. Is also known as adenoviral fiber knob or adenoviral fiber knob with heterologous knob insertions.

**Adjuvant:** Any substance whose admixture with an administered immunogenic determinant / antigen / nucleic acid construct increases or otherwise modifies the immune response to said determinant.

**Amino acid:** Any synthetic or naturally occurring amino carboxylic acid, including any amino acid occurring in peptides and polypeptides including proteins and enzymes synthesized *in vivo* thus including modifications of the amino acids. The term amino acid is herein used synonymously with the term "amino acid residue" which is meant to encompass amino acids as stated which have been reacted with at least one other species, such as 2, for example 3, such as more than 3 other species. The generic term amino acid comprises both natural and non-natural amino acids any of which may be in the "D" or "L" isomeric form. Amino acid may be abbreviated 'aa'.

**Antibody:** Immunoglobulin molecules and active portions of immunoglobulin molecules. Antibodies are for example intact immunoglobulin molecules or fragments thereof retaining the immunologic activity.

**Antigen:** Any substance that can bind to a clonally distributed immune receptor (T-cell or B-cell receptor). Usually a peptide, polypeptide or a multimeric polypeptide. Antigens are preferably capable of eliciting an immune response.

**Boost:** To boost by a booster shot or dose is to give one or more additional doses of an immunizing agent, such as a vaccine, given at a time after an initial dose of a substance used to prime the immune system, to sustain or enhance the immune response elicited by the previous dose of the same (homologous) or another (heterologous) immunizing agent.

**Carrier:** Entity or compound to which antigens are coupled to aid in the induction of an immune response.

**Chimeric protein:** A genetically engineered protein that is encoded by a nucleotide sequence made by a splicing together of two or more complete or partial genes or a series of (non)random nucleic acids.

**Complement:** A complex series of blood proteins whose action "complements" the work of antibodies. Complement destroys bacteria, produces inflammation, and regulates immune reactions.

**Cytokine:** Growth or differentiation modulator, used non-determinative herein, and should not limit the interpretation of the present invention and claims. In addition to the cytokines, adhesion or accessory molecules, or any combination thereof, may be employed alone or in combination with the cytokines.

**CTL:** Cytotoxic T lymphocytes. A sub group of T-cells expressing CD8 along with the T-cell receptor and therefore able to respond to antigens presented by class I molecules.

**Delivery vehicle:** An entity whereby a nucleotide sequence or polypeptide or both can be transported from at least one media to another.

**Fragment:** is used to indicate a non-full length part of a nucleic acid or polypeptide. Thus, a fragment is itself also a nucleic acid or polypeptide, respectively. **Heterologous boost or prime-boost:** wherein the substance used to boost the immune system is different from the substance previously used to prime the immune response.

**Homologous boost or prime-boost:** wherein the substance used to boost the immune system is the same as that previously used to prime the immune response. **Individual:** Any species or subspecies of bird, mammal, fish, amphibian, or reptile, including human beings.

**Invariant chain:** an integral membrane protein glycoprotein that associates with and stabilizes MHC II molecules in the endoplasmatic reticulum and subsequent cellular compartments. Here the term invariant chain covers all naturally occurring or artificially generated full length or fragmented homologous genes and proteins of a certain similarity to human invariant chain. Invariant chain is herein abbreviated li.

**Isolated:** used in connection with nucleic acids, polypeptides, and antibodies disclosed herein 'isolated' refers to these having been identified and separated and/or recovered from a component of their natural, typically cellular, environment. Nucleic acids, polypeptides, and antibodies of the invention are preferably isolated, and vaccines and other compositions of the invention preferably comprise isolated nucleic acids, polypeptides or isolated antibodies.

**MHC:** Major histocompatibility complex, two main subclasses of MHC, Class I and Class II exist.

**Naked DNA:** DNA not associated with histones; often hypomethylated and CpG-rich DNA. Naked DNA may be circular or linear, for example a circular plasmid.

**Nucleic acid:** A chain or sequence of nucleotides that convey genetic information. In regards to the present invention the nucleic acid may be a deoxyribonucleic acid (DNA) or any of the group consisting of ribonucleic acid (RNA), Locked Nucleic Acid (LNA), Peptide Nucleic Acid (PNA), Intercalating nucleic acid (INA), Twisted intercalating nucleic acid (TINA), Hexitol nucleic acids (HNA), arabinonucleic acid (ANA), cyclohexane nucleic acids (CNA), cyclohexenylnucleic acid (CeNA), Glycerol nucleic acid (GNA), threosyl nucleic acid (TNA), Gap-mers, Mix-mers and Morpholinos. **Nucleic acid construct:** A genetically engineered nucleic acid. Typically comprising several elements such as genes or fragments of same, promoters, enhancers, terminators, polyA tails, linkers, polylinkers, operative linkers, multiple cloning sites (MCS), markers, STOP codons, other regulatory elements, internal ribosomal entry sites (IRES) or others.

**Operative linker:** A sequence of nucleotides or amino acid residues that bind together two parts of a nucleic acid construct or (chimeric) polypeptide in a manner securing the biological processing of the nucleic acid or polypeptide.

**Pathogen:** a specific causative agent of disease, especially a biological agent such as a virus, bacteria, prion or parasite that can cause disease to its host, also referred to as an infective agent.

**Peptide:** Plurality of covalently linked amino acid residues defining a sequence and linked by amide bonds. The term is used analogously with oligopeptide and polypeptide. The natural and/or non-natural amino acids may be linked by peptide bonds or by non-peptide bonds. The term peptide also embraces post-translational modifications introduced by chemical or enzyme-catalyzed reactions, as are known in the art. The term can refer to a variant or fragment of a polypeptide.

**Pharmaceutical carriers:** also termed excipients, or stabilizers are non-toxic to the cell or individual being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

**Plurality:** At least two.

**Prime:** Initial priming of the immune system with e.g. DNA to focus the immune response on the required immunogen.

**Prime-boost:** Initial priming of the immune system with e.g. DNA to focus the immune response on the required immunogen, and subsequent boosting of the immune response with a vaccine, leading to an increase in the immune response induced by said vaccine.

**Promoter:** A binding site in a DNA chain at which RNA polymerase binds to initiate transcription of messenger RNA by one or more nearby structural genes.

**Signal peptide:** A short sequence of amino acids that determine the eventual location of a protein in the cell, also referred to as sorting peptide.

**Suitable vaccine:** Any vaccine for use according to the present invention, capable of boosting of the immune response stimulated by the initial priming, characterized in that at least one part of the nucleic acid construct used to prime the immune response and the subsequent vaccine used to boost the immune response are identical. Said at least one identical part of the primer and the booster may be li or a variant thereof, the antigenic peptide or part of the antigenic peptide, or a ubiquitous helper T-cell epitope. **Surfactant:** A surface active agent capable of reducing the surface tension of a liquid in which it is dissolved. A surfactant is a compound containing a polar group which is hydrophilic and a non polar group which is hydrophobic and often composed of a fatty chain.

**Vaccine:** A substance or composition capable of inducing an immune response in an animal: Also referred to as an immunogenic composition in the present text. An immune response being an immune response (humoral/antibody and/or cellular) inducing memory in an organism, resulting in the infectious agent being met by a secondary rather than a primary response, thus reducing its impact on the host organism. A vaccine of the present invention may be given as or prophylactic and/or therapeutic medicament. The composition may comprise one or more of the following: antigen(s), nucleic acid constructs comprising one or more antigens operatively linked to li, carriers, adjuvants and pharmaceutical carriers.

**Variant:** a 'variant' of a given reference nucleic acid or polypeptide refers to a nucleic acid or polypeptide that displays a certain degree of sequence homology/identity to said reference nucleic acid or polypeptide, but is not identical to said reference nucleic acid or polypeptide.

Domain, region and motif may be used interchangeably herein.

### Detailed description of the invention

The present invention relates to a nucleic acid construct comprising sequences encoding invariant chain or a variant thereof operatively linked to at least one antigenic protein or peptide encoding sequences. The nucleic acid construct is used for priming of an immune response, to potentiate the effect of a subsequent booster vaccination.

### The immune response

Vaccines can be used prophylactically: they are given before the actual infection occurs; or therapeutically: where they elicit or accelerate an immune response to a pathogen already in the body. Both methods of vaccination require the establishment of a solid immune response. The immune response that is activated by infection or vaccination depends on the interaction of several cell types, such as T-, B- and antigen presenting cells as well as several different molecules, primarily antigens, MHC molecules, T- and B-cells receptors and many more.

Antigens are peptide fragments presented on the surface of antigen presenting cells by MHC molecules. Antigens can be of foreign, i.e. pathogenic origin, or stem from the organism itself, so called self or auto antigens. The MHC molecules are representatives of a polymorphous gene family encoded by a specific chromosomal region known as the "major histocompatibility complex", hence MHC. Two classes of MHC molecules exist, MHC class I (MHC-I) and MHC class II (MHC-II).

T-helper cells are stimulated by antigens presented by MHC class II (MHC-II) molecules residing on the surface of antigen presenting cells. The MHC-II molecules are synthesized in the endoplasmatic reticulum. During synthesis, they combine with invariant chain (li) in a manner preventing the MHC-II molecules from being loaded with self- or auto-antigens. The MHC-II molecule is by signal sequences in the invariant chain transported to the cell surface in a specific cellular compartment. As the compartment matures by the processing of its contents it progresses from being a lysosome, to a late endosome (after fusion with endocytotic vesicles) to an MHC class II compartment (MIIC). The endocytotic vesicle contains foreign antigen i.e. proteolytically cleaved bacterial peptide fragments. These fragments are by their degradation prepared to be loaded onto the MHC-II molecule. The MHC-II molecule is released by the invariant chain in a two part process when the invariant chain first is degraded proteolytically leaving only a peptide termed CLIP in the MHC-II binding domain, secondly by the removal of CLIP by an HLA-DM molecule. The MHC-II molecule is then free to bind the foreign antigens and present these on the cell surface after fusion of the MIIC vesicle to the plasma membrane. This initiates the humoral immune response as the presented antigen stimulates activation of a T-helper cell which in turn by several means activates a B cell, which ultimately differentiates into an antibody secreting cell.

The cellular immune response is initiated when the T-cell receptor of T-cytotoxic cells recognizes antigen bound to the MHC class I molecule on an antigen presenting cell. MHC-I molecules are not associated with a molecule of a functionality like the invariant chain that associates with MHC-II. The processing of MHC-I into an antigen presenting molecule furthermore differs from that of MHC-II molecules in that the MHC-I molecule is loaded with antigen already in the endoplasmatic reticulum. The antigens presented by the MHC-I molecule are typically peptide fragments cleaved by the proteasome of proteins that have been synthesized by the antigen presenting cell itself. These proteins may be abnormal proteins encoded in the cells own DNA or proteins derived from viruses or other pathogens that have infected the cell and parasitize its protein synthesis machinery. The MHC class I-related proteolytic system is present in virtually all cells.

The functions of the two types of T cells are significantly different, as implied by their names. Cytotoxic T cells eradicate intracellular pathogens and tumors by direct lysis of cells and by secreting cytokines such as γ-interferon. The predominant cytotoxic T cell is the CD8⁺ T cell, which also is antigen specific. Helper T cells also can lyse cells, but their primary function is to secrete cytokines that promote the activities of B cells (antibody-producing cells) and other T cells and thus they broadly enhance the immune response to foreign antigens, including antibody-mediated and cytotoxic T cell-mediated response mechanisms. CD4⁺ T cells are the major helper T cell phenotype in the immune response.

### Nucleic acid construct

An aspect of the present invention relates to nucleic acid constructs such as naked DNA constructs comprising sequences encoding at least one invariant chain or variant thereof operatively linked to at least one antigenic protein or peptide or an antigenic fragment of said protein or peptide, in short an antigen.

In one embodiment, the invention relates to a nucleic acid construct comprising sequences encoding at least one invariant chain or variant thereof operatively linked to at least one antigenic protein or peptide or an antigenic fragment of said protein or peptide, wherein said invariant chain or variant thereof does not comprise the LRMK amino acid residues of the KEY region.

In another embodiment, the invention relates to a nucleic acid construct comprising sequences encoding at least one invariant chain or variant thereof operatively linked to at least one antigenic protein or peptide or an antigenic fragment of said protein or peptide, wherein said invariant chain or variant thereof comprises a variant of the CLIP region.

In another embodiment, the invention relates to a nucleic acid construct comprising sequences encoding at least one invariant chain or variant thereof operatively linked to at least one antigenic protein or peptide or an antigenic fragment of said protein or peptide, wherein said invariant chain or variant thereof does not comprise the first 17 amino acids.

In yet another embodiment, the invention relates to a nucleic acid construct comprising sequences encoding at least one invariant chain or variant thereof operatively linked to at least one antigenic protein or peptide or an antigenic fragment of said protein or peptide, wherein said nucleic acid construct is used for priming of a cancer vaccine.

By nucleic acid construct is understood a genetically engineered nucleic acid. The nucleic acid construct may be a non-replicating and linear nucleic acid, a circular expression vector or an autonomously replicating plasmid. A nucleic acid construct may comprise several elements such as, but not limited to genes or fragments of same, promoters, enhancers, terminators, poly-A tails, linkers, polylinkers, operative linkers, multiple cloning sites (MCS), markers, STOP codons, internal ribosomal entry sites (IRES) and host homologous sequences for integration or other defined elements. It is to be understood that the nucleic acid construct according to the present invention may comprise all or a subset of any combination of the above-mentioned elements.

Methods for engineering nucleic acid constructs are well known in the art (see, e.g., Molecular Cloning: A Laboratory Manual, Sambrook et al., eds., Cold Spring Harbor Laboratory, 2nd Edition, Cold Spring Harbor, N.Y., 1989). Further, nucleic acid constructs according to the present invention may be synthesized without template, and may be obtained from various commercial suppliers (e.g. Genscript Corporation).

The nucleic acid residues comprising the nucleic acid construct may in one embodiment be modified. Said modification may be selected from the group consisting of: acetylation, methylation, phosphorylation, ubiquitination, ribosylation, sulfurization, and others.

The nucleic acid construct according to the present invention may in one embodiment be composed of DNA. In another embodiment, the nucleic acid construct may be composed of a nucleic acid selected from the group consisting of deoxyribonucleic acid (DNA), ribonucleic acid (RNA), Locked Nucleic Acid (LNA), Peptide Nucleic Acid (PNA), Intercalating nucleic acid (INA), Twisted intercalating nucleic acid (TINA), Hexitol nucleic acids (HNA), arabinonucleic acid (ANA), cyclohexane nucleic acids (CNA), cyclohexenylnucleic acid (CeNA), Glycerol nucleic acid (GNA), threosyl nucleic acid (TNA), Gap-mers, Mix-mers, Morpholinos, or a combination thereof.

As discussed above, MHCII-molecules are associated with the invariant chain during processing, until the associated invariant chain is degraded to allow for loading of foreign antigenic peptides onto the MHCII molecules. When applying an 'external' protein construct comprising *invariant chain* - *linker* - *epitope,* wherein the invariant chain comprises the KEY region (comprising LRMK amino acid residues), said protein construct will interact with the MHCII molecule - containing an antigen - at a point when said MHCII molecule is located on the extracellular surface of the cell. Therefore, the effect is external and is depending on the ability of the invariant chain KEY-residue of the protein construct (comprising LRMK amino acid residues) to compete for the loading onto the MHCII-molecules. In other words, the antigen already loaded onto the MHCII-molecule during intracellular processing must be 'tipped off' or removed from the MHCII-molecule on the cellular surface and substituted by the protein construct comprising *invariant chain* - *linker - epitope.* This gives a '1:1'-effect that can not be amplified, and it is invariably dependent on the presence of the LRMK amino acid residues from the invariant chain.

The nucleic acid construct according to the present invention relates to applying an 'internal' nucleic acid construct encoding invariant chain or a variant thereof and also encoding an antigenic peptide or epitope, i.e. the nucleic acid construct is transfected into the intracellular space of cells in a subject. Said nucleic acid construct will use the cellular translational machinery to produce an *invariant chain* - *linker* - *epitope* or *invariant chain* - *epitope* product that will interact with the MHCII molecule or the MHCI molecule - *not* containing an antigen - at a point when said MHC molecule is located inside the cell, such as in an endosome or MIIC. Therefore, the effect is internal and is *not* dependent on the ability of the invariant chain KEY-domain of the protein construct (comprising LRMK amino acid residues) to compete for loading onto the MHC-molecules. Indeed, the effect is not dependent on the presence of the li-KEY region and its LRMK residues. Furthermore, this gives an effect that may be amplified, in that one nucleic acid construct may give rise to more than one product.

The 'internal' use of a nucleic acid construct has several advantages over the 'external' use of a protein construct, as detailed above: 1) Amplification; introduction of a small amount of the nucleic acid construct gives rise to many products that may all bind to the MHC molecules, which also may be secondarily amplified in that said products bound to MHC may be further recycled by internalization to ultimately increase their display, 2) the use of the cells own antigen processing system ensures correct and tight binding of the epitope to the MHC molecule, 3) there is no requirement for the LRMK residues of the li-KEY region and the native li-CLIP domain.

### Codon-optimization and Degenerate nucleic acid sequences

The expression of functional proteins in heterologous hosts is the cornerstone of modern biotechnology. Unfortunately, many proteins are difficult to express outside their original contexts. They may contain expression-limiting regulatory elements, come from organisms that use non-canonical nucleotide codes or from a gene rife with codons rarely used in the desired host. Improvements in the speed and efficiency of gene synthesis have rendered feasible complete gene redesign for maximum protein expression. For example, protein expression can improve dramatically when the codon frequency of the gene under study is matched to that of the host expression system. For example, a redesign strategy may include not only the use of optimum codon biases, but also the alteration of mRNA structural elements and the modification of translation and initiation regions. Techniques for codon optimization are known to the person skilled in the art, and may be performed by commercial suppliers such as GenScript Corporation.

It is understood, that the nucleic acid construct comprising invariant chain or a variant thereof according to the present invention may be codon-optimized in any way so as to produce - by translation into protein i.e. amino acids - an amino acid sequence comprising an invariant chain that corresponds to the amino acid sequence disclosed in SEQ ID NO: 2 (human li), or variants thereof according to the present invention.

Likewise, the nucleic acid construct comprising invariant chain according to the present invention may be codon-optimized in any way so as to produce - by translation into protein i.e. amino acids - an amino acid sequence comprising an invariant chain that corresponds to the amino acid sequence of any animal in which the nucleic acid construct may be used to prime an immune response; including any vertebrate, mammal, fish or bird; or variants thereof according to the present invention.

*Codon bias:* Codon bias has been identified as the single most important factor in prokaryotic gene expression. The degree to which a given codon appears in the genetic code varies significantly between organisms, between proteins expressed at high and low levels and even between different portions of the same operon. The reason for this is almost certainly because preferred codons correlate with the abundance of cognate tRNAs available within the cell. This relationship serves to optimize the translational system and to balance codon concentration with isoacceptor tRNA concentration.

*Replace infrequently used codons:* In general, the more rare codons that a gene contains, the less likely it is that the heterologous protein will be expressed at a reasonable level within that specific host system. These levels become even lower if the rare codons appear in clusters or in the N-terminal portion of the protein. Replacing rare codons with others that more closely reflect the host system's codon bias without modifying the amino acid sequence can increase the levels of functional protein expression.

*Eliminate problematic codons:* Any codon that an organism uses less than 5% to 10% of the time may cause problems, regardless of where it is from. Again, close or adjacent codons can have more affect on protein expression than they could separately. Eliminating rare codons and codons that could be read as termination signals can prevent cases of low or nonexistent expression.

*Express viral proteins in mammalian hosts:* Even viral genes can be successfully expressed in mammalian cell lines if the gene is properly prepared. Viral genes' dense information loads frequently result in overlapping reading frames. Many viral genes also encode *cis*-acting negative regulatory sequences within the coding sequence. Viral genes can be resynthesized not only to express only the desired protein but also to disrupt regulatory elements, thereby enhancing protein production. Viral codon optimization is especially useful in DNA vaccine research because it increases the immunogenicity of the target.

*Other constraints:* Although codon bias plays a large role in gene expression, the choice of expression vectors and transcriptional promoters is also important. The nucleotide sequences surrounding the N-terminal region of the protein are particularly sensitive, both to the presence of rare codons and to the identities of the codons immediately adjacent to the initiation AUG. There is also some interplay between translation and mRNA stability.

### Degeneracy of the genetic code

It follows from the above that the genetic code has redundancy but no ambiguity. For example, although codons GAA and GAG both specify glutamic acid (redundancy), neither of them specifies any other amino acid (no ambiguity) (see the codon table below for the full correlation). The codons encoding one amino acid may differ in any of their three positions. The degeneracy of the genetic code is what accounts for the existence of silent mutations. Degeneracy results because a triplet code of four bases designates 20 amino acids and a stop codon.

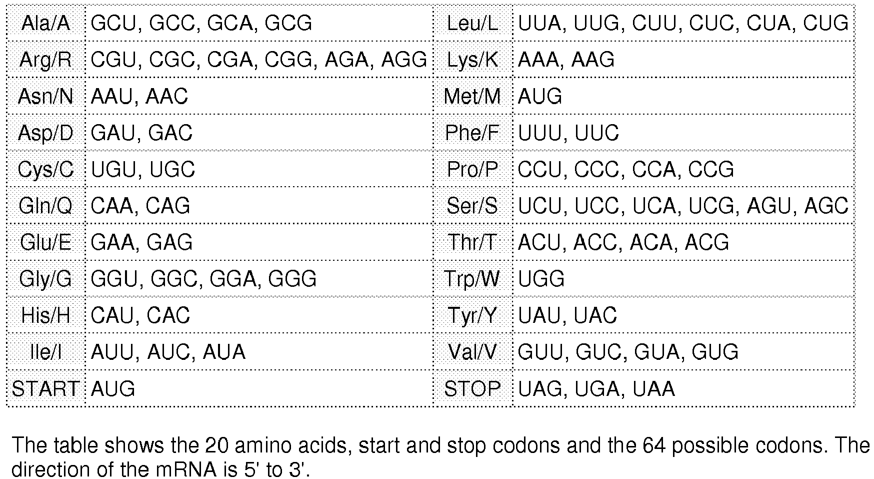

### Synonymous substitution

Silent mutations or substitutions are DNA mutations that do not result in a change to the amino acid sequence of a protein. They may occur in a non-coding region (outside of a gene or within an intron), or they may occur within an exon in a manner that does not alter the final amino acid sequence. The phrase silent mutation or substitution is often used interchangeably with the phrase synonymous mutation or substitution; however, synonymous mutations or substitutions are a subcategory of the former, occurring only within exons.

It is understood, that the nucleic acid construct comprising invariant chain or a variant thereof according to the present invention may comprise a synonymous substitution so as to produce - by translation into protein i.e. amino acids - an amino acid sequence comprising an invariant chain that corresponds to the amino acid sequence disclosed in SEQ ID NO: 2 (human li), or variants thereof according to the present invention.

Likewise, the nucleic acid construct comprising invariant chain according to the present invention may comprise a synonymous substitution so as to produce - by translation into protein i.e. amino acids - an amino acid sequence comprising an invariant chain that corresponds to the amino acid sequence of any animal in which the nucleic acid construct may be used to prime an immune response; including any vertebrate, mammal, fish or bird; or variants thereof according to the present invention.

### Non-synonymous substitution into synonymous amino acids

A non-synonymous substitution causes a change in the amino acid. However, amino acids are grouped according to the properties of said amino acid, and the substitution of one amino acid with another amino acid may have no impact of the function or properties of the protein comprising said amino acid if the substitution results in a synonymous amino acid. Such substitutions may be denoted conservative substitution or mutation: A change in a DNA or RNA sequence that leads to the replacement of one amino acid with a biochemically similar one.

It is thus understood, that the nucleic acid construct comprising invariant chain or a variant thereof according to the present invention may comprise a non-synonymous substitution so as to produce - by translation into protein i.e. amino acids - an amino acid sequence comprising a variant of invariant chain, wherein said non-synonymous substitution results in the substitution of one or more amino acids which are synonymous.

Synonymous substitutions may comprise substitution of a hydrophobic amino acid with another hydrophobic amino acid; substitution of a hydrophilic amino acid with another hydrophilic amino acid; substitution of a polar amino acid with another polar amino acid; substitution of a non-polar amino acid with another non-polar amino acid; substitution of a positively charged amino acid with another positively charged amino acid; substitution of a negatively charged amino acid with another negatively charged amino acid; substitution of a neutral amino acid with another neutral amino acid; substitution of an ambiguous amino acid with its counterpart ambiguous charged amino acid such as isoleucine and leucine, asparagine and aspartic acid and glutamine and glutamic acid; substitution of an aromatic amino acid with another aromatic amino acid; substitution of an aliphatic amino acid with another aliphatic amino acid; or the substitution of any amino acid with alanine. These substitutions may be denoted equal-value substitution.

### Splice variants

Alternative splicing is the RNA splicing variation mechanism in which the exons of the primary gene transcript, the pre-mRNA, are separated and reconnected so as to produce alternative ribonucleotide arrangements. These linear combinations then undergo the process of translation where specific and unique sequences of amino acids are specified, resulting in isoform proteins or splice variants. In this way, alternative splicing uses genetic expression to facilitate the synthesis of a greater variety of proteins. In eukaryotes, alternative splicing is an important step towards higher efficiency, because information can be stored much more economically. Several proteins can be encoded in a DNA sequence whose length would only be enough for two proteins in the prokaryote way of coding.

The nucleic acid construct of the present invention may in one embodiment be designed so as to give rise to multiple antigenic peptides of fragments of antigenic peptides and/or multiple invariant chains or variants thereof.

In one embodiment, the nucleic acid construct according to the present invention comprises at least 1, such as 2, for example 3, such as 4, for example 5, such as 6, for example 7, such as 8, for example 9, such as 10, for example 11, such as 12, for example 13, such as 14, for example 15, such as 16, for example 17 such as 18, for example 19, such as 20 splice variants of an antigenic peptide or a fragment of said antigenic peptide.

The more than one antigenic peptide splice variants may encompass identical or non-identical antigenic peptides.

In another embodiment, the nucleic acid construct according to the present invention comprises at least 1, such as 2, for example 3, such as 4, for example 5, such as 6, for example 7, such as 8, for example 9, such as 10, for example 11, such as 12, for example 13, such as 14, for example 15, such as 16, for example 17 such as 18, for example 19, such as 20 splice variants of invariant chain or variants thereof.

The more than one invariant chain splice variant may encompass identical or non-identical invariant chain or variants thereof.

In one embodiment, at least one splice variant of invariant chain comprises native full length invariant chain. In another embodiment, at least one splice variant of invariant chain comprises a variant of invariant chain. In yet another embodiment, at least one splice variant of invariant chain comprises a variant of invariant chain wherein said li does not comprise the LRMK amino acid residues of the li-KEY region. In another embodiment, at least one splice variant of invariant chain comprises a variant of invariant chain wherein said li does not comprise the M91 and M99 residues of the CLIP domain.

It follows that the splice variant may comprise any combination of identical or non-identical antigenic peptides and/or identical or non-identical invariant chain or variants thereof.

In this manner it is possible to 'shuffle' sequences (exons) comprising different domains or regions of invariant chain, so as to obtain variants of invariant chain by alternative splicing. In this manner it is also possible to 'shuffle' sequences (exons) comprising different domains or regions of the antigenic peptide(s), so as to obtain variants of said antigenic peptide(s) by alternative splicing.

### Invariant chain

The invariant chain (li) or MHC class II associated invariant chain or CD74 or p31, is a non-polymorphic type II integral membrane protein, see SEQ ID NOs: 2 and 4 for the amino acid sequences of human and mouse li, respectively, and likewise SEQ ID NOs: 1 and 3 for the nucleic acid sequences of human and mouse li, respectively. Invariant chain has multiple functions in lymphocyte maturation and in adaptive immune responses, in particular targeting to lysosomal compartments were the li CLIP sequence can occupy MHC class II molecules until these are fused with endosomal compartments (Pieters J. 1997, Curr. Opin. Immunol., 9:8996). Additionally li has been shown to function as an MHC class I chaperone (Morris et al, 2004, Immunol. Res. 30:171-179) and by its endosomal targeting sequence, to facilitate proliferation of CD4⁺, but not CD8⁺ T-cells directed against covalently linked antigen (Diebold et al., 2001, Gene Ther. 8:487-493).

The invariant chain protein comprises several domains: a cytosolic domain which includes a signal or sorting peptide (also known as the lysosomal targeting sequence), a transmembrane domain, and a luminal domain which in itself comprises a CLIP region, KEY region (comprising the LRMK residues), core domain and trimerization domain. Both of these domains are flanked by highly flexible regions (Strumptner-Cuvelette & Benaroch, 2002, Biochem. Biophys. Acta., 1542:1-13). Invariant chain has been characterized in several organisms, including vertebrates (e.g. chicken), mammals (e.g. cow, dog, mouse and rat) and human.

The present invention relates to nucleic acid constructs comprising sequences wherein at least one invariant chain or variant thereof is organism specific or can be related to a specific organism. Preferably, at least one invariant chain is of vertebrate origin, more preferably of mammalian origin and most preferably of human origin. In relation hereto the sequence defined by SEQ ID NO: 1 is the nucleic acid sequence of the invariant chain from human. In another preferred embodiment, at least one invariant chain is of avian origin, most preferred from Gallus gallus domesticus (chicken). In yet another preferred embodiment, at least one invariant chain is derived from fish, most preferred from fish which may be bred in a fish farm (such as salmon or trout). In yet another preferred embodiment, at least one invariant chain is derived from a ferret.

The employed invariant chain is preferably the invariant chain of the organism that is to receive the nucleic acid construct. It is an object of the present invention that the invariant chain and the host organisms or receivers of the treatment are of the same species.

In one embodiment of the invention, the nucleic acid construct comprising at least one invariant chain or variant thereof is with the proviso that when the nucleic acid construct comprises a variant of at least one invariant chain, said invariant chain does not comprise the LRMK amino acid residues of the li-KEY sequence.

In another embodiment of the invention, the nucleic acid construct comprising at least one invariant chain or variant thereof is with the proviso that when the nucleic acid construct comprises a variant of at least one invariant chain, said invariant chain comprises a variant of the li-CLIP domain. Said variant is in one embodiment a substitution of methionine at positions 91 and 99 with another amino acid. Said variant is in another embodiment a double M91A M99A point mutation (substitution of the amino acid methionine to alanine at positions 91 and 99).

In another embodiment, the li variant is a deletion the first 17 amino acids of li (Δ17li).

In a third embodiment, the li variant comprises both a substitution of methionine at positions 91 and 99 and a deletion the first 17 amino acids of li.

The inventors have surprisingly found, that the LRMK residues if the li-KEY domain are not essential for priming of an immune response according to the present invention.

Also, the inventors have surprisingly found that the substitution of methionine at positions 91 and 99 of li increases the MHCII presentation.

Furthermore, the inventors have found that deleting the first 17 amino acids of li surprisingly increases the memory response.

The inventors have further found that a central part of the invariant chain comprising residues number 50 to 118 is essential for obtaining the full effect of li. This variant of li lacks a trimerization domain. Thus, in one embodiment the nucleic acid construct comprises at least one invariant chain or variant thereof wherein said invariant chain comprises amino acid residues number 50 to 118 coupled to a trimerization domain from another protein. Said other protein may for example be a bacterial protein or an adenoviral fiber protein.

The present invention also relates to a nucleic acid construct wherein the encoded at least one invariant chain is a fragment of the sequence identified in SEQ ID NO: 2 of at least 40 amino acids and of at least 85% identity to the same fragment of SEQ ID NO: 2.

The fragment is a fragment of at least 40 amino acids from any part of the invariant chain as set forth in SEQ ID NO: 2. This includes a fragment including residues 1 to 40, 10 to 50, 20 to 60, 25 to 65, 30 to 70, 35 to 75, 40 to 80, 45 to 85, 50 to 90, 55 to 95, 60 to 100, 65 to 105, 70 to 110, 75 to 115, 80 to 120, 85 to 125, 90 to 130, 95 to 135, 100 to 140, 105 to 145, 110 to 150, 115 to 155, 120 to 160, 125 to 165, 130 to 170, 135 to 175, 140 to 180, 145 to 185, 150 to 190, 155 to 195, 160 to 200,165 to 205, 170 to 210 and 175 to 216. It also includes fragments as any of the above listed expanding up to 5 residues to either side hereof. It further includes fragment of at least 50 residues, of at least 60 residues, of at least 70 residues, of at least 80 residues, of at least 90 residues, of at least 100 residues, of at least 110 residues, of at least 120 residues, of at least 130 residues, of at least 140 residues, of at least 150 residues, of at least 160 residues, of at least 170 residues, of at least 180 residues of at least 190 residues, of at least 200 residues and of at least 210 residues.

Any of the above described fragments of at least 85 % sequence identity, for example at least 90 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence identity with SEQ ID NO: 2 are included within the scope of the present invention.

The identity/homology between amino acid sequences may be calculated using well known scoring matrices such as any one of BLOSUM 30, BLOSUM 40, BLOSUM 45, BLOSUM 50, BLOSUM 55, BLOSUM 60, BLOSUM 62, BLOSUM 65, BLOSUM 70, BLOSUM 75, BLOSUM 80, BLOSUM 85, and BLOSUM 90.

In one embodiment the present invention is a nucleic acid construct wherein the encoded at least one invariant chain is a fragment of SEQ ID NO: 2 of at least 186 amino acids. This includes any of the fragments as defined above, and which thus share identity with the sequence of the invariant chain of SEQ ID NO: 2.

The present invention furthermore relates to a nucleic acid construct wherein the encoded at least one invariant chain is at least 85% identical to SEQ ID NO: 2.

This encompasses that any sequence derived from the invariant chain as put forward in SEQ ID NO: 2 of at least 85 % sequence identity, for example at least 90 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence homology with SEQ ID NO: 2 are included within the scope of the present invention. This includes sequences that are either longer or shorter than the sequence described in SEQ ID NO: 2.

Any of the above described sequences regardless of origin, sequence identity or length are from hereon termed variants of invariant chain.

It follows, that it is within the scope of the present invention that a variant of invariant chain from any organism may be a variant according to the above, i.e. that the variant may be altered in the li-KEY region and/or be altered in the li-CLIP-region and/or be a fragment of the invariant chain of an organism and/or be at least 85% identical to said invariant chain either over all the sequence of the invariant chain or within the fragment of same. The invariant chain may also be from a related species of organism or be from a distantly related species.

Another aspect of the present invention relates to the addition, removal or substitution of regions, peptides or domains of the at least one invariant chain as encoded by the nucleic acid construct. The removal of one or more of these regions, peptides or domains will truncate the resulting invariant chain. The addition or replacement of a region, peptide or domain includes the options of choosing these sequences from known sources such as naturally occurring proteins or polypeptides or from artificially synthesized polypeptides or nucleic acid residues encoding the same. The addition of regions, domains or peptides includes the option of adding one, two or more of each type or of different types of regions, domains, peptides and one, two, three or more of the nucleic acids encoding these regions, domains and peptides. These may be identical or differ from one another based on the sequence. The regions, peptides and domains need not arise from the same organism as the scaffold invariant chain.

The removal of regions, domains or peptides includes the option of removing one, two, three or more of each type or of different types of regions, domains, peptides and removing one, two, three, four, five, six, seven, eight, nine, ten, eleven, twelve, thirteen, fourteen, fifteen, sixteen, seventeen, eighteen, nineteen, twenty or more of the amino acid residues encoding these regions, domains and peptides. It is well known in the art to perform additions, deletions and substitutions of individual as well as stretches of nucleotides which will encode the resulting polypeptide.

Aligning nucleic acid and especially protein sequences of homologous genes or proteins from different organisms can be of great assistance when determining which substitutions, deletions, rearrangements or other alterations it would be beneficial to construct. Aligning human and murine invariant chain sequences as illustrated below, gives an indication of which amino acid residues may be of importance for the structure and function of the invariant chain in these organisms - these are the residues which are conserved between the two sequences. Likewise, the presumably less important residues are the ones in which the sequences differ. It is of interest in regard to the present invention to perform substitutions and/or deletions of the variant residues / regions. When attempting to mutate or delete or otherwise alter the sequence of e.g. the human invariant chain in order to improve its immune response stimulating capacity, it may also be relevant to examine the conserved residues and make e.g. homologous substitutions (i.e. substitutions where the amino acids are considered to be of e.g. same structural quality, polarity, hydrophobicity or other).

The LRMK amino acid residues of the KEY regions are underlined in the below alignment of the invariant chain protein derived from human and mouse.

| | | |
|---|---|---|
| human | 1 | MDDQRDLISNNEQLPMLGRRPGAPESKCSRALYTGFSILVTLLLAGQATTAYFLYQQG |
| murine | 1 | MDDQRDLISNHEQLPILGNRPREPE-RCSRGALYTGVSVLVALLLAGQATTAYFLYQQQG |
| | | |
| human | 61 | RLDKLTVTSQNLQLENLRMKLPKPPKPVSKMRMATPLLMQALPMGALPQGPMQNATKYGN |
| murine | 60 | RLDKLTITSQLESLRMKLPKSAKPVSQMRMATPLLMRPMSMDNMLLGPVKNVTKYGN |
| | | |
| human | 121 | MTEDHVMHLLQNADPLKVYPPLKGSFPENLRHLKNTMETIDWKVFESWMHHWLLFEMSRH |
| murine | 120 | MTQDHVMHLLTRSGPLE-YPQLKGTFPENLKHLKNSMDGVNWKIFESWMKQWLLFEMSKN |
| | | |
| human | 181 | SLEQK-PTDAPPKESLELEDPSSGLGVTKQDLGPVPM |
| murine | 179 | SLLEEKKPTEAPPKEPLDMEDLSSGLGVTRQELGQVTL |

### Key region

One preferred embodiment of the present invention relates to the removal, substitution, or replacement of the KEY region of the at least one invariant chain. As described above, the addition or replacement of the KEY region includes the options of adding or replacing the existing KEY region in the variant of the invariant chain or chains chosen, with KEY regions from invariant chains of the same or other organisms or of variants of KEY regions from the same or other organisms. The variant KEY regions may, as follows from the above, be specifically generated mutant versions of the KEY region, generated by single or multiple nucleic acid substitutions, deletions or additions. A preferred embodiment comprises alone the N-terminally or C-terminally adjacent sequences to the KEY region but without the KEY region itself. By adjacent is meant any amino acids within 10 residues of the KEY region, within 20 residues, within 30 residues, within 40 residues, within 50 residues, within 75 residues or within 100 residues of the KEY region.

A most preferred embodiment comprises one or more substitutions or deletions of the KEY region, resulting in the substitution or deletion of one, two, three or four amino acid residues of the LRMK amino acids comprised in the KEY region. In one embodiment, at least one, such as two, for example three, such as four of the LRMK amino acids comprised in the KEY region are deleted. In another embodiment, at least one, such as two, for example three, such as four of the LRMK amino acids comprised in the KEY region are substituted by other amino acids. Said amino acids may be any amino acid selected from the group consisting of: G (glycine), P (proline), A (alanine), V (valine), L (leucine), I (isoleucine), M (methionine), C (cysteine), F (phenylalanine), Y (tyrosine), W (tryptophan), H (histidine), K (lysine), R (arginine), Q (glutamine), N (asparagine), E (glutamic acid), D (aspartic acid), S (serine) and T (threonine).

In one particular embodiment, the LRMK amino acid residues are each substituted with alanine (A) amino acid residues, thus the sequence reads: AAAA. In another embodiment, the LRMK amino acid residues are substituted with amino acids that comprise synonymous or equal-value substitutions. For example, amino acid residue L may be substituted with I, V, M or F; R may be substituted with K, H, E or D; M may be substituted with L, I, F or V; and K may be substituted with H or R.

In one embodiment of the present invention, the KEY region may comprise more than the LRMK residues, or the LRMK residues may be replaced with a sequence of more than four amino acid residues.

An embodiment of the present invention relates to fragments of invariant chain as described above without the KEY region. These fragments may be at least 5 amino acid residues long, at least 10 residues, at least 15 residues, at least 20 residues, at least 25 residues, at least 30 residues or at least 35 residues in length. Another embodiment relates to fragments of invariant chain wherein the signal peptide is removed and the invariant chain fragment is at least 10 amino acid residues long, at least 15 residues, at least 20 residues, at least 25 residues, at least 30 residues, at least 35 residues, at least 50 residues at least 60 residues, at least 70 residues at least 80 residues, at least 90 residues, at least 100 residues, at least 110 residues at least 120 residues at least 130 residues, at least 140 residues, at least 150 residues, at least 160 residues, at least 170 residues, or at least 180 residues in length.

In one embodiment of the invention, the at least one invariant chain encoded by the nucleic acid construct as described herein does not comprise the LRMK amino acid residues of the li-KEY region.

In one embodiment, the present invention thus relates to a nucleic acid construct comprising at least one invariant chain or variant thereof, linked to at least one antigenic protein or peptide or an antigenic fragment of said protein or peptide, wherein said invariant chain or variant thereof does not comprise the LRMK amino acid residues of the li-KEY region.

### CLIP region

Another embodiment of the present invention relates to the removal, addition, or replacement of the CLIP region of the at least one invariant chain. As described above, the addition or replacement of the CLIP region includes the options of adding or replacing the existing CLIP region in the variant of the invariant chain or chains chosen, with CLIP regions from invariant chains of the same or other organisms or of variants of CLIP regions from the same or other organisms. The variant CLIP regions may, as follows from the above, be specifically generated mutant versions of the CLIP region, generated by single or multiple nucleic acid substitutions, deletions or additions. A preferred embodiment comprises the CLIP region alone, or the CLIP region together with the N-terminally adjacent sequence or the C-terminally adjacent sequence without any other regions or domains of invariant chain. Other preferred embodiments comprise alone the N-terminally or C-terminally adjacent sequences to the CLIP region but without the CLIP region itself. By adjacent is meant any amino acids within 10 residues of the CLIP region, within 20 residues, within 30 residues, within 40 residues, within 50 residues, within 75 residues or within 100 residues of the CLIP region.

A preferred embodiment comprises one or more substitutions or deletions of the CLIP region, resulting in the substitution or deletion of one, two, three, four or more amino acid residues of the CLIP region. In one embodiment, at least one, such as two, for example three, such as four or more of the amino acids comprised in the CLIP region are deleted. In another embodiment, at least one, such as two, for example three, such as four or more of the amino acids comprised in the CLIP region are substituted by other amino acids. Said amino acids may be any amino acid selected from the group consisting of: G (glycine), P (proline), A (alanine), V (valine), L (leucine), I (isoleucine), M (methionine), C (cysteine), F (phenylalanine), Y (tyrosine), W (tryptophan), H (histidine), K (lysine), R (arginine), Q (glutamine), N (asparagine), E (glutamic acid), D (aspartic acid), S (serine) and T (threonine).

An embodiment of the present invention relates to fragments of invariant chain as described above without the CLIP region. These fragments may be at least 5 amino acid residues long, at least 10 residues, at least 15 residues, at least 20 residues, at least 25 residues, at least 30 residues or at least 35 residues in length. Another embodiment relates to fragments of invariant chain wherein the signal peptide is removed and the invariant chain fragment is at least 10 amino acid residues long, at least 15 residues, at least 20 residues, at least 25 residues, at least 30 residues, at least 35 residues, at least 50 residues at least 60 residues, at least 70 residues at least 80 residues, at least 90 residues, at least 100 residues, at least 110 residues at least 120 residues at least 130 residues, at least 140 residues, at least 150 residues, at least 160 residues, at least 170 residues, or at least 180 residues in length.

In one particular embodiment, the M amino acid residues on positions 91 and 99 are each substituted with alanine (A) amino acid residues, thus the sequence reads: M91A M99A. In another embodiment, the M amino acid residues on positions 91 and 99 are substituted with amino acids that comprise synonymous or equal-value substitutions.

In one embodiment of the invention, the at least one invariant chain encoded by the nucleic acid construct as described herein does not comprise the M amino acid residues on positions 91 and 99 of the li-CLIP sequence.

In one embodiment, the present invention thus relates to a nucleic acid construct comprising at least one invariant chain or variant thereof, linked to at least one antigenic protein or peptide or an antigenic fragment of said protein or peptide, wherein said invariant chain or variant thereof does not comprise the M amino acid residues on positions 91 and 99 of the li-CLIP region.

### N- or C- terminal alterations:

One embodiment of the present invention relates to the removal (deletion), substitution, or replacement of the N- or C- terminal regions of the at least one invariant chain. As described above, the addition or replacement of the N- or C- terminal regions includes the options of adding or replacing the existing N- or C- terminal regions in the variant of the invariant chain or chains chosen, with N- or C- terminal regions from invariant chains or other proteins of the same or other organisms or of variants of N- or C-terminal regions from the same or other organisms. The variant N- or C- terminal regions may, as follows from the above, be specifically generated mutant versions of the N- or C- terminal regions, generated by single or multiple nucleic acid substitutions, deletions or additions.

An embodiment comprises the deletion of the first (N-terminal) or the last (C-terminal) amino acids of the li, such as the first or last 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 125, 130, 135, 140, 145 or 150 amino acids of li.

In one particular embodiment of the present invention, the li variant comprises a deletion the first 17 amino acids of li (Δ17li).

An embodiment of the present invention relates to fragments of invariant chain as described above without the complete N- or C- terminal regions. These fragments may be at least 5 amino acid residues long, at least 10 residues, at least 15 residues, at least 20 residues, at least 25 residues, at least 30 residues or at least 35 residues in length. Another embodiment relates to fragments of invariant chain wherein the signal peptide is removed and the invariant chain fragment is at least 10 amino acid residues long, at least 15 residues, at least 20 residues, at least 25 residues, at least 30 residues, at least 35 residues, at least 50 residues at least 60 residues, at least 70 residues at least 80 residues, at least 90 residues, at least 100 residues, at least 110 residues at least 120 residues at least 130 residues, at least 140 residues, at least 150 residues, at least 160 residues, at least 170 residues, or at least 180 residues in length.

In one embodiment of the present invention, all or part of the transmembrane segment of li may be replaced with the corresponding segment from any other protein, such as the chemokine receptor CCR6 TM6.

In another embodiment of the present invention, all or part of the transmembrane segment of li may be replaced with the corresponding segment from the chemokine receptor CCR6 TM6.

### Signal peptide

Another embodiment of the present invention relates to the at least one invariant chain wherein the signal peptide is removed, replaced or added onto the sequence encoding the invariant chain. A signal peptide is a short sequence of amino acids that determine the eventual location of a protein in the cell, also referred to as a sorting peptide. Signal peptides that determine the location of proteins to subcellular compartments such as the endoplasmatic reticulum, golgi apparatus and the various compartments comprising the golgi apparatus, the nucleus, the plasma membrane, mitochondria and the various spaces and membranes herein, peroxisomes, lysosomes, endosomes and secretory vesicles among others are all included within the scope of the present invention. A preferred embodiment comprises alone the lysosomal targeting sequence of invariant chain.

### Antigen

Any of the above variants of invariant chain are encompassed in the present invention in the form wherein at least one of said variants is operatively linked to at least one antigen such as an antigenic protein or peptide or an antigenic fragment of said protein or peptide.

It is an object of the present invention to include but not limit the antigenic proteins or peptides or fragments of said proteins or peptides to stem from pathogenic organisms, cancer-specific polypeptides and antigens, and proteins or peptides associated with an abnormal physiological response.

More preferably it is an object of the present invention to include an antigen originating from any of the following types of pathogens: virus, micro organisms and parasites. This includes pathogens of any animal known. It is preferable to have an antigen from a mammalian pathogen i.e. a pathogen that specifically targets mammalian animals such as a ferret. It is preferred to have an antigen from a human pathogen. In general, any antigen that is found to be associated with a human pathogen or disease may be used.

In another embodiment, it is preferable to have an antigen from an avian pathogen i.e. a pathogen that specifically targets birds or fowls. It is more preferred to have an antigen from a chicken (gallus gallus domesticus). In general, any antigen that is found to be associated with an avian pathogen may be used.

In yet another embodiment, it is preferable to have an antigen from a piscine pathogen i.e. a pathogen that specifically targets fish. It is more preferred to have an antigen from a fish that may be bred in a fish farm. In general, any antigen that is found to be associated with a piscine pathogen may be used.

### Viral antigens

In a preferred embodiment at least one antigen may originate from, but is not limited to any of the following families of virus: Adenovirus, arenaviridae, astroviridae, bunyaviridae, caliciviridae, coronaviridae, flaviviridae, herpesviridae, orthomyxoviridae, paramyxoviridae, picornaviridae, poxviridae, reoviridae, retroviridae, rhabdoviridae and togaviridae.

More specifically at least one antigen or antigenic sequence may be derived from any of the following virus: Influenza A such as H1N1, H1N2, H3N2 and H5N1 (bird flu), Influenza B, Influenza C virus, Hepatitis A virus, Hepatitis B virus, Hepatitis C virus, Hepatitis D virus, Hepatitis E virus, Rotavirus, any virus of the Norwalk virus group, enteric adenoviruses, parvovirus, Dengue fever virus, Monkey pox, Mononegavirales, Lyssavirus such as rabies virus, Lagos bat virus, Mokola virus, Duvenhage virus, European bat virus 1 & 2 and Australian bat virus, Ephemerovirus, Vesiculovirus, Vesicular Stomatitis Virus (VSV), Herpesviruses such as Herpes simplex virus types 1 and 2, varicella zoster, cytomegalovirus, Epstein-Bar virus (EBV), human herpesvirusses (HHV), human herpesvirus type 6 and 8, Human immunodeficiency virus (HIV), papilloma virus, murine gammaherpesvirus, Arenaviruses such as Argentine hemorrhagic fever virus, Bolivian hemorrhagic fever virus, Sabia-associated hemorrhagic fever virus, Venezuelan hemorrhagic fever virus, Lassa fever virus, Machupo virus, Lymphocytic choriomeningitis virus (LCMV), Bunyaviridiae such as Crimean-Congo hemorrhagic fever virus, Hantavirus, hemorrhagic fever with renal syndrome causing virus, Rift Valley fever virus, Filoviridae (filovirus) including Ebola hemorrhagic fever and Marburg hemorrhagic fever, Flaviviridae including Kaysanur Forest disease virus, Omsk hemorrhagic fever virus, Tick-borne encephalitis causing virus and Paramyxoviridae such as Hendra virus and Nipah virus, variola major and variola minor (smallpox), alphaviruses such as Venezuelan equine encephalitis virus, eastern equine encephalitis virus, western equine encephalitis virus, SARS-associated coronavirus (SARS-CoV), West Nile virus, any encephaliltis causing virus.

In a preferred embodiment of the invention the at least one antigenic protein or peptide is from a virus selected from the group of: HIV, Hepatitis C virus, influenza virus, herpes virus, Lassa, Ebola, smallpox, Bird flu, filovirus, Marburg, and papilloma virus.

In a more preferred embodiment of the invention the at least one antigenic protein or peptide is selected from the group of and/or may be at least one antigenic fragment of any of the following: vesicular stomatitis virus glycoprotein (VSV-GP); Influenza A NS-1 (non-structural protein 1), M1 (matrix protein 1), NP (nucleoprotein), NEP, M2, M2e, HA, NA, PA, PB1, PB2, PB1-F2; LCMV NP, LCMV GP; Ebola GP, Ebola NP; HIV antigens tat, vif, rev, vpr, gag, pol, nef, env, vpu; SIV antigens tat, vif, rev, vpr, gag, pol, nef, env; murine gammaherpesvirus M2, M3 and ORF73 (such as MHV-68 M2, M3 and ORF73); chicken Ovalbumin (OVA); or a helper T-cell epitope. It is within the scope of the invention to combine two or more of any of the herein mentioned antigens.

### Microorganism antigens

An embodiment of the present invention includes at least one antigenic protein or peptide or fragment of an antigenic protein or peptide from a micro organism. More specifically at least one antigen may be derived from the one of the following from a non-exhaustive list: Anthrax (Bacillus anthracis), Mycobacterium tuberculosis, Salmonella (Salmonella gallinarum, S. pullorum, S. typhi, S. enteridtidis, S. paratyphi, S. dublin, S. typhimurium), Clostridium botulinum, Clostridium perfringens, Corynebacterium diphtheriae, Bordetella pertussis, Campylobacter such as Campylobacter jejuni, Crytococcus neoformans, Yersinia pestis, Yersinia enterocolitica, Yersinia pseudotuberculosis, Listeria monocytogenes, Leptospira species, Legionella pneumophila, Borrelia burgdorferi, Streptococcus species such as Streptococcus pneumoniae, Neisseria meningitides, Haemophilus influenzae, Vibrio species such as Vibrio cholerae O1, V. cholerae non-O1, V. parahaemolyticus, V. parahaemolyticus, V. alginolyticus, V. furnissii, V. carchariae, V. hollisae, V. cincinnatiensis, V. metschnikovii, V. damsela, V. mimicus, V. fluvialis, V. vulnificus, Bacillus cereus, Aeromonas hydrophila, Aeromonas caviae, Aeromonas sobria & Aeromonas veronii, Plesiomonas shigelloides, Shigella species such as Shigella sonnei, S. boydii, S. flexneri, and S. dysenteriae, Enterovirulent Escherichia coli EEC (Escherichia coli - enterotoxigenic (ETEC), Escherichia coli - enteropathogenic (EPEC), Escherichia coli O157:H7 enterohemorrhagic (EHEC), Escherichia coli - enteroinvasive (EIEC)), Staphylococcus species, such as S. aureus and especially the vancomycin intermediate/resistant species (VISA/VRSA) or the multidrug resistant species (MRSA), Shigella species, such as S. flexneri, S. sonnei, S. dysenteriae, Cryptosporidium parvum, Brucella species such as B. abortus, B. melitensis, B.ovis, B. suis, and B. canis, Burkholderia mallei and Burkholderia pseudomallei, Chlamydia psittaci, Coxiella burnetii, Francisella tularensis, Rickettsia prowazekii, Histoplasma capsulatum, Coccidioides immitis.

In a preferred embodiment of the invention the at least one antigenic protein or peptide is from a micro-organism selected from the group of: Mycobacterium tuberculosis, Bacillus anthracis, Staphylococcus species, and Vibrio species.

### Parasitic antigen

An embodiment of the invention relates to a nucleic acid construct, wherein the at least one antigenic protein or peptide encoded is from a parasite.

Another embodiment of the present invention relates to a nucleic acid construct comprising combinations of at least two antigenic proteins or peptides from any of the abovementioned pathogens.

Preferably the antigen is derived from, but not limited to, a parasite selected from the group of: Plasmodium species such as Plasmodium malariae, Plasmodium ovale, Plasmodium vivax, Plasmodium falciparum, Endolimax nana, Giardia lamblia, Entamoeba histolytica, Cryptosporidum parvum, Blastocystis hominis, Trichomonas vaginalis, Toxoplasma gondii, Cyclospora cayetanensis, Cryptosporidium muris, Pneumocystis carinii, Leishmania donovani, Leishmania tropica, Leishmania braziliensis, Leishmania mexicana, Acanthamoeba species such as Acanthamoeba castellanii, and A. culbertsoni, Naegleria fowleri, Trypanosoma cruzi, Trypanosoma brucei rhodesiense, Trypanosoma brucei gambiense, Isospora belli, Balantidium coli, Roundworm (Ascaris lumbricoides), Hookworm (Necator Americanus, Ancylostoma duodenal), Pinworm (Enterobius vermicularis), Roundworm (Toxocara canis, Toxocara cati), Heart worm (Dirofilaria immitis), Strongyloides (Stronglyoides stercoralis), Trichinella (Trichinella spiralis), Filaria (Wuchereria bancrofti, Brugia malayi, Onchocerca volvulus, Loa loa, Mansonella streptocerca, Mansonella perstans, Mansonella ozzardi), and Anisakine larvae (Anisakis simplex (herring worm), Pseudoterranova (Phocanema, Terranova) decipiens (cod or seal worm), Contracaecum species, and Hysterothylacium (Thynnascaris species) Trichuris trichiura, Beef tapeworm (Taenia saginata), Pork tapeworm (Taenia solium), Fish tapeworm (Diphyllobothrium latum), and Dog tapeworm (Dipylidium caninum), Intestinal fluke (Fasciolopsis buski), Blood fluke (Schistosoma japonicum, Schistosoma mansoni) Schistosoma haematobium), Liver fluke (Clonorchis sinensis), Oriental lung fluke (Paragonimus westermani), and Sheep liver fluke (Fasciola hepatica), Nanophyetus salmincola and N. schikhobalowi.

In a preferred embodiment of the invention the at least one antigenic protein or peptide is from a parasite selected from the group of: Plasmodium species, Leishmania species, and Trypanosoma species.

The at least one antigen of the present invention may be Var2Csa from Plasmodium falciparum. In a preferred embodiment of the invention, the at least one antigenic protein or peptide or fragment of an antigenic protein or peptide is Var2Csa.

### Domestic animal antigen

An aspect of the present invention relates to antigens and/or antigenic sequences derived from diseases or agents that infect domestic animals, especially commercially relevant animals such as pigs, cows, horses, sheep, goats, llamas, rabbits, mink, mice, rats, dogs, cats, ferrets, poultry such as chicken, turkeys, pheasants and others, fish such as trout, salmon, cod and other farmed species. Examples of diseases or agents here of from which at least one antigen or antigenic sequence may be derived include, but are not limited to: Multiple species diseases such as: Anthrax, Aujeszky's disease, Bluetongue, Brucellosis such as: Brucella abortus, Brucella melitensis or Brucella suis; Crimean Congo haemorrhagic fever, Echinococcosis/hydatidosis, virus of the family Picornaviridae, genus Aphthovirus causing Foot and Mouth disease especially any of the seven immunologically distinct serotypes: A, O, C, SAT1, SAT2, SAT3, Asia1, or Heartwater, Japanese encephalitis, Leptospirosis, New world screwworm (Cochliomyia hominivorax), Old world screwworm (Chrysomya bezziana), Paratuberculosis, Q fever, Rabies, Rift Valley fever, Rinderpest, Trichinellosis, Tularemia, Vesicular stomatitis or West Nile fever; Cattle diseases such as: Bovine anaplasmosis, Bovine babesiosis, Bovine genital campylobacteriosis, Bovine spongiform encephalopathy, Bovine tuberculosis, Bovine viral diarrhoea, Contagious bovine pleuropneumonia, Enzootic bovine leukosis, Haemorrhagic septicaemia, Infectious bovine rhinotracheitis / infectious pustular vulvovaginitis, Lumpky skin disease, Malignant catarrhal fever, Theileriosis, Trichomonosis or Trypanosomosis (tsetse-transmitted); Sheep and goat diseases such as: Caprine arthritis / encephalitis, Contagious agalactia, Contagious caprine pleuropneumonia, Enzootic abortion of ewes (ovine chlamydiosis), Maedi-visna, Nairobi sheep disease, Ovine epididymitis (Brucella ovis), Peste des petits ruminants, Salmonellosis (S. abortusovis), Scrapie, Sheep pox and goat pox; Equine diseases such as: African horse sickness, Contagious equine metritis, Dourine, Equine encephalomyelitis (Eastern), Equine encephalomyelitis (Western), Equine infectious anaemia, Equine influenza, Equine piroplasmosis, Equine rhinopneumonitis, Equine viral arteritis, Glanders, Surra (Trypanosoma evansi) or Venezuelan equine encephalomyelitis; Swine diseases such as: African swine fever, Classical swine fever, Nipah virus encephalitis, Porcine cysticercosis, Porcine reproductive and respiratory syndrome, Swine vesicular disease or Transmissible gastroenteritis; Avian diseases such as: Avian chlamydiosis, Avian infectious bronchitis, Avian infectious laryngotracheitis, Avian mycoplasmosis (M. gallisepticum), Avian mycoplasmosis (M. synoviae), Duck virus hepatitis, Fowl cholera, Fowl typhoid, Highly pathogenic avian influenza this being any Influenzavirus A or B and especially H5N1, Infectious bursal disease (Gumboro disease), Marek's disease, Newcastle disease, Pullorum disease or Turkey rhinotracheitis; Lagomorph and rodent diseases such as: Virus enteritis, Myxomatosis or Rabbit haemorrhagic disease; Fish diseases such as: Epizootic haematopoietic necrosis, Infectious haematopoietic necrosis, Spring viraemia of carp, Viral haemorrhagic septicaemia, Infectious pancreatic necrosis, Infectious salmon anaemia, Epizootic ulcerative syndrome, Bacterial kidney disease (Renibacterium salmoninarum), Gyrodactylosis (Gyrodactylus salaris), Red sea bream iridoviral disease; or other diseases such as Camelpox or Leishmaniosis.

In a preferred embodiment of the invention the at least one antigenic protein or peptide is from Aujeszky's disease, Foot and mouth disease, Vesicular stomatitis virus, Avian influenza or Newcastle disease.

Yet a preferred embodiment of the present invention relates to the at least one antigenic protein or peptide or fragment of said antigenic protein or peptide being an antigenic peptide or protein with at least 85% identity to any of the above described antigens. The homology or identity between amino acids may be calculated by any of the previously mentioned BLOSUM scoring matrices.

### Cancer antigens

Many protein/glycoproteins have been identified and linked to certain types of cancer; these are referred to as cancer-specific polypeptides, tumor-associated antigens or cancer antigens. In general, any antigen that is found to be associated with cancer tumors may be used. One way in which cancer-specific antigens may be found is by subtraction analyses such as various microarray analyses, such as DNA microarray analysis. Herein the gene-expression pattern (as seen in the level of RNA or protein encoded by said genes) between healthy and cancerous patients, between groups of cancerous patients or between healthy and cancerous tissue in the same patient is compared. The genes that have approximately equal expression levels are "subtracted" from each other leaving the genes / gene products that differ between the healthy and cancerous tissue. This approach is known in the art and may be used as a method of identifying novel cancer antigens or to create a gene-expression profile specific for a given patient or group of patients. Antigens thus identified, both single antigen and the combinations in which they may have been found fall within the scope of the present invention.

Preferably the at least one antigen of the present invention is derived from, but not limited to, a cancer-specific polypeptide selected from the group of: MAGE-3, MAGE-1, gp100, gp75, TRP-2, tyrosinase, MART-1, CEA, Ras, p53, B-Catenin, gp43, GAGE-1, BAGE-1, PSA, MUC-1, 2, 3, and HSP-70, TRP-1, gp100/pmel17, beta-HCG, Ras mutants, p53 mutants, HMW melanoma antigen, MUC-18, HOJ-1, cyclin-dependent kinase 4 (Cdk4), Caspase 8, HER-2/neu, Bcr-Abl tyrosine kinase, carcinoembryonic antigen (CEA), telomerase, SV40 Large T, Human papilloma virus HPV type 6, 11, 16, 18, 31 and 33; HPV derived viral oncogene E5, E6, E7 and L1; Survivin, Bcl-XL, MCL-1 and Rho-C.

In a preferred embodiment of the invention, the at least one antigenic protein or peptide or fragment of an antigenic protein or peptide is from a cancer-specific polypeptide selected from the group of: HPV derived viral oncogene E5, E6, E7 and L1; Survivin, Bcl-XL, MCL-1 and Rho-C.

### Antigen associated with an abnormal physiological response

An embodiment of the invention relates to a nucleic acid construct, wherein the at least one antigenic protein or peptide or fragment of an antigenic protein or peptide is from a polypeptide associated with an abnormal physiological response. Such an abnormal physiological response includes, but is not limited to autoimmune diseases, allergic reactions, cancers and congenital diseases. A non-exhaustive list of examples hereof includes diseases such rheumatoid arthritis, systemic lupus erythematosus, multiple sclerosis, psoriasis and Crohn's disease.

### Operative linker

An aspect of the present invention relates to the nucleic acid construct wherein the operative link between the invariant chain and the antigenic protein or peptide or fragment of antigenic protein or peptide either is a direct link or a link mediated by a spacer region. By the term operative linker is understood a sequence of nucleotides or amino acid residues that bind together two parts of a nucleic acid construct or chimeric polypeptide in a manner securing the biological processing of the nucleic acid or polypeptide. If the operative linker is a direct link, the two nucleic acids each encoding either an open reading frame or a fragment of an open reading frame are placed immediately adjacent to each other and thereby also in frame. If the operative linker is mediated by a spacer region, a series of nucleotides are inserted between the nucleotides encoding the at least one invariant chain and the at least one antigenic peptide, respectively. It is within the scope of the present invention having a spacer region wherein the spacer region merely is a series of nucleotides linking the at least two elements of the present invention in a manner retaining the open reading frames, or the spacer region may encode one or more signals or separate elements as defined herein below.

In one particular embodiment the invention comprises an operative linker, wherein the operative linker is a spacer region.

In one embodiment the invention comprises a spacer region encoding at least one helper epitope for class II MHC molecules. An example of a helper epitope is an immunogenic determinant such as Diphtheria toxin. Especially Diphtheria toxin B fragment COOH-terminal region has been shown to be immunogenic in mice. Furthermore, HSP70, in part or in whole, as well as other immunogenic peptides, such as influenza viral or immunogenic sequences or peptides with an anchoring motif to HLA class I and class II molecules, also may be encoded in the spacer region of the nucleic acid construct.

In another embodiment the spacer region of the nucleic acid construct encodes at least one protease cleavage site. Cleavage sites of lysosomal proteases such as cathepsins, aspartate proteases and zinc proteases as well as other intracellular proteases fall within the scope of the present invention.

In yet an embodiment the operative linker of the nucleic acid construct may comprise at least one siRNA or miRNA encoding sequence. siRNAs (small interfering RNAs) and miRNAs (microRNAs) target endogenous RNAs, in a sequence-specific manner, for degradation. An siRNA or miRNA encoded within the nucleic acid construct of the present invention may thus be chosen to target an undesirable gene product.

In another embodiment the operative linker comprises at least one polylinker or multiple cloning site (MCS). Polylinkers and MCS's are series of nucleotides comprising restriction enzyme recognition sequences, i.e. sites where a restriction enzyme cut the DNA in blunt or staggered manner facilitating the subcloning of other fragments / sequences of DNA into the nucleic acid construct. The recognition sequences of the polylinkers / MCS's are typically unique meaning that they are not found elsewhere on the nucleic acid construct. The operative linker may furthermore comprise one or more stop or termination codons that signal the release of the nascent polypeptide from the ribosome. The operative linker may also comprise at least one IRES (Internal Ribosomal Entry Site) and / or at least one promoter. An IRES is a nucleotide sequence that allows for translation initiation in the middle of a messenger RNA (mRNA) sequence as part of the greater process of protein synthesis. A promoter is a DNA sequence that enables a gene to be transcribed. The promoter is recognized by RNA polymerase, which then initiates transcription, see in the below. The promoter may be single or bidirectional.

In one embodiment the operative linker spanning the region between the invariant chain and the at least one antigen is an operative linker comprising at least one polylinker, and at least one promoter, and optionally also at least one IRES. These elements may be placed in any order. In a further preferred embodiment, the STOP codon of the invariant chain has been deleted, and the polylinker has been cloned into the vector in a manner conserving the open reading frame allowing for in frame reading of the at least one antigen that is inserted into the polylinker. This has the advantage of facilitating subcloning of multiple antigens into the same construct in one step or in multiple cloning steps and allowing for the simultaneous expression of multiple antigens in the same frame as the invariant chain. A STOP codon may be inserted after the polylinker for translation termination. This embodiment may be combined with any of the above helper epitopes, mi/siRNAs or any of the other elements herein described.

An embodiment of the present invention relates to the placement of the operative linker in relations to the at least one invariant chain and the at least one antigenic protein or peptide or fragment of said protein or peptide, wherein the at least one antigenic peptide encoding sequences are placed: within the invariant chain sequence, at the front end of the invariant chain sequence, at the terminal part of the invariant chain sequence. This is done in a manner ensuring the readability of the open reading frame of the construct, so that the antigenic peptide is: preceded, surrounded or rounded off by, at least one operative linker.

Another embodiment of the present invention further relates to the placement of the operative linker in relations to the at least one invariant chain and the at least one antigenic protein or peptide or fragment of said protein or peptide, wherein the at least one antigenic peptide encoding sequence preferably is placed at the terminal part of the invariant chain and an operative linker is inserted herein between: The terminal part being the first or last residue of the invariant chain or fragment hereof.

In another embodiment, the nucleic acid construct does not comprise an operative linker; rather, the at least one antigenic peptide encoding sequence is tethered directly to the invariant chain. The at least one antigenic peptide encoding sequences may be placed: within the invariant chain sequence, at the front end of the invariant chain sequence, at the terminal part of the invariant chain sequence. This is done in a manner ensuring the readability of the open reading frame of the construct,

There are advantages to both possibilities of including or excluding an operative linker; excluding the linker may in one embodiment reduce the possibility of priming an immune response against said linker rather than priming an immune against the antigenic peptide.

### Combinations

It is within the scope of the present invention that the nucleic acid construct encodes a plurality of elements. The elements being the at least one invariant chain or variant thereof and the at least one antigenic protein or peptide or fragment of said protein or peptide. It therefore falls within the scope of the present invention to have a plurality of invariant chains or variants thereof each of these being operatively linked to each other and to a plurality of antigenic proteins or peptides or fragments of antigenic proteins or peptides, wherein these also are operatively linked. The elements of the nucleic acid construct must thus be operatively linked to each other. Several series of invariant chains or variants thereof each operatively linked to one antigenic protein or peptide or fragment of said protein or peptide, each of these series being operatively linked to each other are encompassed within the present invention.

Advantages and very important aspects of the present invention relate to the fact that any type of immune response e.g. T cell mediated and antibody mediated responses, can be initiated, both with epitopes known to be weak antigens, with polypeptides of unknown antigenic properties, and with multiple epitopes/antigens simultaneously.

It is therefore also within the scope of the present invention that a preferred embodiment is a nucleic acid construct encoding at least one invariant chain or variant thereof operatively linked to a plurality of antigenic proteins or peptides or fragment of proteins or peptides, such as two, three, four, five, six, eight, ten, twelve or more antigenic proteins or peptides or fragment of proteins or peptides.

The nucleic acid construct may comprise additional elements. These include but are not limited to: internal ribosomal entry sites (IRES); genes encoding proteins related to antigen presentation such as LAMP, calreticulin, Hsp 33, Hsp 60, Hsp70, Hsp90, Hsp100, sHSP (small heat shock protein) and heat shock binding proteins such as 77-residue DNAJ-homologous Hsp73-binding domain; genes encoding proteins that are related to intracellular spreading such as VP22, HIV Tat, Cx43 or other connexins and intercellular gap-junction constituents; genes encoding natural killer cell (NK-cell) activation molecules such as H60 and cytokines, chicken ovalbumin, or any T-helper cell epitope.

In a preferred embodiment of the present invention the nucleic acid construct comprises at least one gene encoding a protein related to antigen presentation such as LAMP, LIMP, calreticulin Hsp 33, Hsp 60, Hsp70, Hsp90, Hsp100, sHSP (small heat shock protein) or 77-residue DNAJ-homologous Hsp73-binding domain.

In yet a preferred embodiment of the present invention the nucleic acid construct comprises at least one gene encoding a protein related to intracellular spreading such as VP22, Cx43, HIV Tat, other connexins or intercellular gap-junction constituents.

### Promoter

The term promoter will be used here to refer to a group of transcriptional control modules that are clustered around the initiation site for RNA polymerase II. Much of the thinking about how promoters are organized derives from analyses of several viral promoters, including those for the HSV thymidine kinase (tk) and SV40 early transcription units. These studies, augmented by more recent work, have shown that promoters are composed of discrete functional modules, each consisting of approximately 7-20 bp of DNA, and containing one or more recognition sites for transcriptional activator proteins. At least one module in each promoter functions to position the start site for RNA synthesis. The best known example of this is the TATA box, but in some promoters lacking a TATA box, such as the promoter for the mammalian terminal deoxynucleotidyl transferase gene and the promoter for the SV 40 late genes, a discrete element overlying the start site itself helps to fix the place of initiation.

Additional promoter elements regulate the frequency of transcriptional initiation. Typically, these are located in the region 30-110 bp upstream of the start site, although a number of promoters have recently been shown to contain functional elements downstream of the start site as well. The spacing between elements is flexible, so that promoter function is preserved when elements are inverted or moved relative to one another. In the tk promoter, the spacing between elements can be increased to 50 bp apart before activity begins to decline. Depending on the promoter, it appears that individual elements can function either cooperatively or independently to activate transcription. Any promoter that can direct transcription initiation of the sequences encoded by the nucleic acid construct may be used in the invention.

An aspect of the present invention comprises the nucleic acid construct wherein the at least one operatively linked invariant chain and antigenic protein or peptide encoding sequence is preceded by a promoter enabling expression of the construct.

It is a further aspect that the promoter is selected from the group of constitutive promoters, inducible promoters, organism specific promoters, tissue specific promoters, cell type specific promoters and inflammation specific promoters.

Examples of promoters include, but are not limited to: constitutive promoters such as: simian virus 40 (SV40) early promoter, a mouse mammary tumor virus promoter, a human immunodeficiency virus long terminal repeat promoter, a Moloney virus promoter, an avian leukaemia virus promoter, an Epstein-Barr virus immediate early promoter, a Rous sarcoma virus (RSV) promoter, a human actin promoter, a human myosin promoter, a human haemoglobin promoter, cytomegalovirus (CMV) promoter and a human muscle creatine promoter, inducible promoters such as: a metallothionine promoter, a glucocorticoid promoter, a progesterone promoter, and a tetracycline promoter (tet-on or tet-off), tissue specific promoters such as: HER-2 promoter and PSA associated promoter and bidirectional promoters, that are capable of initiating transcription in either direction from the promoter.

Advantages of using an inducible promoter includes the option of providing a "dormant" nucleic acid construct that can be activated at will. This may be of use if the priming of an immune response preferably only is induced locally vs. systemically within a body (e.g. in cases involving cancer), or the priming of an immune response is detrimental to the health of the recipient at the time of administration.

In a preferred embodiment the nucleic acid construct comprises a promoter selected from the group of: CMV promoter, SV40 promoter and RSV promoter.

### Delivery vehicle

An aspect of the present invention comprises the nucleic acid construct as described in any of the above, comprised within a delivery vehicle. A delivery vehicle is an entity whereby a nucleotide sequence or polypeptide or both can be transported from at least one media to another. Delivery vehicles are generally used for expression of the sequences encoded within the nucleic acid construct and/or for the intracellular delivery of the construct or the polypeptide encoded therein.

The nucleic acid construct may be transferred into cells in vivo or ex vivo; the latter by removing the target tissue (i.e., liver cells or white blood cells) from the patient, transferring the construct in vitro and then replanting the transduced cells into the patient.

Methods of non-viral delivery include physical (carrier-free delivery) and chemical approaches (synthetic vector-based delivery).

Physical approaches, including needle injection, gene gun, jet injection, electroporation, ultrasound, and hydrodynamic delivery, employ a physical force that permeates the cell membrane and facilitates intracellular gene transfer. Said physical force may be electrical or mechanical.

The chemical approaches use synthetic or naturally occurring compounds as carriers to deliver the transgene into cells. The most frequently studied strategy for non-viral gene delivery is the formulation of DNA into condensed particles by using cationic lipids or cationic polymers. The DNA-containing particles are subsequently taken up by cells via endocytosis, macropinocytosis, or phagocytosis in the form of intracellular vesicles, from which a small fraction of the DNA is released into the cytoplasm and migrates into the nucleus, where transgene expression takes place.

It is within the scope of the present invention that the delivery vehicle is a vehicle selected from the group of: RNA based vehicles, DNA based vehicles/ vectors, lipid based vehicles, polymer based vehicles and virally derived DNA or RNA vehicles.

A preferred embodiment of the present invention regards delivery of the nucleic acid construct by mechanical or electrical techniques.
- Physical
   ∘ Injection: One preferred embodiment regards simple injection of the nucleic acid construct in solution. Injection is normally conducted intramuscularly (IM) in skeletal muscle, intradermally (ID) or to the liver, with the nucleic acid construct being delivered to the extracellular spaces. Delivery by injection can be assisted by electroporation; by using hypertonic solutions of saline or sucrose; by temporarily damaging muscle fibers with myotoxins such as bupivacaine; or by adding substances capable of enhancing the efficiency of DNA internalization by target cells such as transferrin, water.immiscible solvents, non-ionic polymers, surfactants or nuclease inhibitors.
   ∘ Gene gun: The gene gun or the Biolistic Particle Delivery System is a device for injecting cells with genetic information. The payload is an elemental particle of a heavy metal such as gold, silver or tungsten coated with e.g. plasmid DNA. This technique is often simply referred to as biolistics. Compressed helium may be used as the propellant. Especially the coating of the nucleic acid construct upon gold particles, such as colloidal gold particles, is a favoured embodiment.
   ∘ Pneumatic (Jet) Injection: No particles required, aqueous solution
   ∘ Electroporation, or electropermeabilization, is a significant increase in the electrical conductivity and permeability of the cell plasma membrane caused by an externally applied electrical field.
   ∘ Ultrasound-Facilitated Gene Transfer: ultrasound creates membrane pores and facilitates intracellular gene transfer through passive diffusion of DNA across the membrane pores. The efficiency can be enhanced by the use of contrast agents or conditions that make membranes more fluidic.
   ∘ Hydrodynamic Gene Delivery: Hydrodynamic gene delivery is a simple method that introduces naked plasmid DNA into cells in highly perfused internal organs (eg, the liver). In rodents, rapid tail vein injection of a large volume of DNA solution causes a transient overflow of injected solution at the inferior vena cava that exceeds the cardiac output. As a result, the injection induces a flow of DNA solution in retrograde into the liver, a rapid rise of intrahepatic pressure, liver expansion, and reversible disruption of the liver fenestrae.
- Chemical
   ∘ Cationic Lipid-Mediated Gene Delivery: Although some cationic lipids alone exhibit good transfection activity, they are often formulated with a noncharged phospholipid or cholesterol as a helper lipid to form liposomes. Upon mixing with cationic liposomes, plasmid DNA is condensed into small quasi-stable particles called lipoplexes. DNA in lipoplexes is well protected from nuclease degradation. Lipoplexes are able to trigger cellular uptake and facilitate the release of DNA from the intracellular vesicles before reaching destructive lysosomal compartments.
   ∘ Cationic Polymer-Mediated Gene Transfer: most cationic polymers share the function of condensing DNA into small particles and facilitating cellular uptake via endocytosis through charge-charge interaction with anionic sites on cell surfaces. Cationic polymer DNA carriers include polyethylenimine (PEI), polyamidoamine and polypropylamine dendrimers, polyallylamine, cationic dextran, chitosan, cationic proteins (polylysine, protamine, and histones), and cationic peptides.
   ∘ Lipid-Polymer Hybrid System: DNA precondensed with polycations, then coated with either cationic liposomes, anionic liposomes, or amphiphilic polymers with or without helper lipids.

Examples of chemical delivery vehicles include, but are not limited to: biodegradable polymer microspheres, lipid based formulations such as liposome carriers, cationically charged molecules such as liposomes, calcium salts or dendrimers, lipopolysaccharides, polypeptides and polysaccharides.

Alternative physical delivery methods may include aerosol instillation of a naked nucleic acid construct on mucosal surfaces, such as the nasal and lung mucosa; topical administration of the nucleic acid construct to the eye and mucosal tissues; and hydration such as stromal hydration by which saline solution is forced into the corneal stroma of the eye.

Another embodiment of the present invention comprises a vector which herein is denoted a viral vector (i.e. not a virus) as a delivery vehicle. Viral vectors according to the present invention are made from a modified viral genome, i.e. the actual DNA or RNA forming the viral genome, and introduced in naked form. Thus, any coat structures surrounding the viral genome made from viral or non-viral proteins are not part of the viral vector according to the present invention.

The virus from which the viral vector is derived is selected from the non-exhaustive group of: adenoviruses, retroviruses, lentiviruses, adeno-associated viruses, herpesviruses, vaccinia viruses, foamy viruses, cytomegaloviruses, Semliki forest virus, poxviruses, RNA virus vector and DNA virus vector. Such viral vectors are well known in the art.

### Recombinant cell

An aspect of the present invention relates to a cell comprising the nucleic acid construct as defined in any of the above. Such a recombinant cell can be used a tool for in vitro research, as a delivery vehicle for the nucleic acid construct or as part of a gene-therapy regime. The nucleic acid construct according to the invention can be introduced into cells by techniques well known in the art and which include microinjection of DNA into the nucleus of a cell, transfection, electroporation, lipofection/liposome fusion and particle bombardment. Suitable cells include autologous and non-autologous cells, and may include xenogenic cells.

In a preferred embodiment the nucleic acid construct of the present invention is comprised within an antigen presenting cell (APC). Any cell that presents antigens on its surface in association with an MHC molecule is considered an antigen presenting cell. Such cells include but are not limited to macrophages, dendritic cells, B cells, hybrid APCs, and foster APCs. Methods of making hybrid APCs are well known in the art.

In a more preferred embodiment the APC is a professional antigen presenting cell and most preferably the APC is an MHC-I and/or MHC-II expressing cell.

The APC according to any of the above may be a stem cell obtained from a patient. After introducing the nucleic acid construct of the invention, the stem cell may be reintroduced into the patient in an attempt to treat the patient of a medical condition. Preferably, the cell isolated from the patient is a stem cell capable of differentiating into an antigen presenting cell.

It is furthermore included within the scope of the present invention that the antigen presenting cell comprising the nucleic acid construct of the present invention does not express any co-stimulatory signals and the antigenic protein or peptide or antigenic fragment of said protein or peptide is an auto-antigen.

### Chimeric proteins and antibodies

An object of the present invention is the chimeric protein encoded by the nucleic acid constructs as described herein above, comprising at least one operatively linked invariant chain or variants thereof and at least one antigenic protein or peptide or fragment of said antigenic protein or peptide. By chimeric protein is understood a genetically engineered protein that is encoded by a nucleotide sequence made by splicing together of two or more complete or partial genes or a series of (non)random nucleic acids.

An aspect of the present invention relates to an antibody that can recognize the chimeric protein as defined herein above. By the term antibody is understood immunoglobulin molecules and active portions of immunoglobulin molecules. Antibodies are for example intact immunoglobulin molecules or fragments thereof retaining the immunologic activity. Such antibodies can be used for the passive immunization of an animal, or for use in an assay for detecting proteins to which the antibody binds.

### Nucleic acid construct compositions

An aspect of the present invention relates to a composition comprising a nucleic acid sequence encoding at least one invariant chain or variants thereof operatively linked to at least one antigenic protein or peptide or fragment of said antigenic protein or peptide. The composition may thus comprise a nucleic acid construct as defined in any of the above. The composition may furthermore be used as a medicament.

The nucleic acid construct composition according to the invention can be formulated according to known methods such as by the admixture of one or more pharmaceutically acceptable carriers, also known as excipients or stabilizers with the active agent. These excipients may be acceptable for administration to any individual / animal, preferably to vertebrates and more preferably to humans as they are non-toxic to the cell or individual being exposed thereto at the dosages and concentrations employed. Often the physiologically acceptable carrier is an aqueous pH buffered solution. Examples of such excipients, carriers and methods of formulation may be found e.g. in Remington's Pharmaceutical Sciences (Maack Publishing Co, Easton, PA). Examples of physiologically acceptable carriers include but are not limited to: buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

To formulate a pharmaceutically acceptable composition suitable for effective administration, such compositions will according to the invention contain an effective amount of the nucleic acid construct, the nucleic acid construct comprised within a delivery vehicle or the chimeric protein encoded within the nucleic acid construct as described herein. Often, if priming the immune response with protein or polypeptides as encoded by the nucleic acid construct of the present invention, a carrier will be used as a scaffold by coupling the proteins or peptides hereto and thus aiding in the induction of an immune response. The carrier protein may be any conventional carrier including any protein suitable for presenting immunogenic determinants. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Immunisation of the animal may be carried out with adjuvants and/or pharmaceutical carriers. Conventional carrier proteins include, but are not limited to, keyhole limpet hemocyanin, serum proteins such as transferrin, bovine serum albumin, or human serum albumin, an ovalbumin, immunoglobulins, or hormones, such as insulin. The carrier may be present together with an adjuvant or independently here from.

In the following, nucleic acid construct composition or composition are meant to encompass compositions useful for prophylactic and therapeutic use, including stimulating an immune response in a patient. It is further contemplated that the composition of the invention does not induce any systemic or local toxicity reactions or any other side effects.

In one preferred embodiment, the phrase 'composition' as used herein refers to a composition for priming an immune response.

In a preferred embodiment the nucleic acid construct is packaged. Packaging means for the nucleic acid construct include means selected from, but not limited to the group of: RNA based or DNA based vectors, lipid based carriers, viral expression vectors, viral delivery vectors, coating of colloidal gold particles and biodegradable polymer microspheres. Any of the previously mentioned delivery means may thus be used for packing purposes for use in a composition.

In one embodiment the packaging means of the nucleic acid construct is a viral expression vector selected from, but not limited to the group of: adenovirus, retrovirus, lentivirus, adeno-associated virus, herpes virus, vaccinia virus and DNA virus vector. The viral vector may be a replication deficient or conditionally replication deficient viral vector.

An aspect of the invention relates to a composition comprising at least two vectors. This encompasses that any one or two different nucleic acid constructs as described may be packed into at least two vectors, these vectors being of a type as described in any of the above. The invention furthermore relates to a composition comprising three, four, five or six vectors. Again, these vectors may differ from one another or not, and may carry identical or different nucleic acid constructs as described herein above.

A further aspect of the present invention relates to a composition comprising at least one chimeric protein as encoded by any of the nucleic acid constructs described herein. When a chimeric protein or polypeptide is to be used as an immunogen, it may be produced by expression of any one or more of the nucleic acid constructs described above in a recombinant cell or it may be prepared by chemical synthesis by methods known in the art. As described in the above, such chimeric proteins and / or peptides may be coupled to carriers to increase the immunologic response to the proteins / peptides and may be administered with or without an adjuvant and/or excipient.

In one embodiment, the present invention relates to the use of the nucleic acid construct as described herein for the production of a composition.

### Enhancing an immune response: Traditional adjuvants

Adjuvants may be included in the composition to enhance the specific immune response. Thus, it is particular important to identify an adjuvant that when combined with the antigen(s) / nucleic acid constructs and / or delivery vehicles (any of which may also be referred to as immunogenic determinant), results in a composition capable of inducing a strong specific immunological response. The immunogenic determinant may also be mixed with two or more different adjuvants prior to immunisation. Compositions are also referred to as immunogenic compositions in the present text.

A large number of adjuvants have been described and used for the generation of antibodies in laboratory animals, such as mouse, rats and rabbits. In such setting the tolerance of side effect is rather high as the main aim is to obtain a strong antibody response. For use and for approval for use in pharmaceuticals, and especially for use in humans it is required that the components of the composition, including the adjuvant, are well characterized. It is further required that the composition has minimal risk of any adverse reaction, such as granuloma, abscesses or fever.

An embodiment of the present invention relates to a composition comprising an adjuvant. In a preferred embodiment the composition is suitable for administration to a mammal, such as a human being. Therefore the preferred adjuvant is suitable for administration to a mammal and most preferably is suitable for administration to a human being.

In another preferred embodiment the composition is suitable for administration to a bird or a fish, and most preferably to a chicken (Gallus gallus domesticus). Therefore the preferred adjuvant is suitable for administration to a bird or a fish.

The choice of adjuvant may further be selected by its ability to stimulate the type of immune response desired, B-cell or/and T-cell activation and the composition may be formulated to optimize distribution and presentation to the relevant lymphatic tissues.

Adjuvants pertaining to the present invention may be grouped according to their origin, be it mineral, bacterial, plant, synthetic, or host product. The first group under this classification is the mineral adjuvants, such as aluminum compounds. Antigens precipitated with aluminum salts or antigens mixed with or adsorbed to performed aluminum compounds have been used extensively to augment immune responses in animals and humans. Aluminium particles have been demonstrated in regional lymph nodes of rabbits seven days following immunization, and it may be that another significant function is to direct antigen to T cell containing areas in the nodes themselves. Adjuvant potency has been shown to correlate with intimation of the draining lymph nodes. While many studies have confirmed that antigens administered with aluminium salts lead to increased humoral immunity, cell mediated immunity appears to be only slightly increased, as measured by delayed-type hypersensitivity. Aluminium hydroxide has also been described as activating the complement pathway. This mechanism may play a role in the local inflammatory response as well as immunoglobulin production and B cell memory. Furthermore, aluminum hydroxide can protect the antigen from rapid catabolism. Primarily because of their excellent record of safety, aluminum compounds are presently the only adjuvants used in humans. Another large group of adjuvants is those of bacterial origin. Adjuvants with bacterial origins can be purified and synthesized (e.g. muramyl dipeptides, lipid A) and host mediators have been cloned (Interleukin 1 and 2). The last decade has brought significant progress in the chemical purification of several adjuvants of active components of bacterial origin: Bordetella pertussis, Mycobacterium tuberculosis, lipopoly-saccharide, Freund's Complete Adjuvant (FCA) and Freund's Incomplete Adjuvant (Difco Laboratories, Detroit, Mich.) and Merck Adjuvant 65 (Merck and Company, Inc., Rahway, N.J.). Additionally suitable adjuvants in accordance with the present invention are e.g. Titermax Classical adjuvant (SIGMA-ALDRICH), ISCOMS, Quil A, ALUN, see US 58767 and 5,554,372, Lipid A derivatives, choleratoxin derivatives, HSP derivatives, LPS derivatives, synthetic peptide matrixes, GMDP, and other as well as combined with immunostimulants (US 5,876,735). B. pertussis is of interest as an adjuvant in the context of the present invention due to its ability to modulate cell-mediated immunity through action on T-lymphocyte populations. For lipopolysaccharide and Freund's Complete Adjuvant, adjuvant active moieties have been identified and synthesized which permit study of structure-function relationships. These are also considered for inclusion in immunogenic compositions according to the present invention.

Lipopolysaccharide (LPS) and its various derivatives, including lipid A, have been found to be powerful adjuvants in combination with liposomes or other lipid emulsions. It is not yet certain whether derivatives with sufficiently low toxicity for general use in humans can be produced. Freund's Complete Adjuvant is the standard in most experimental studies.

Mineral oil may be added to the immunogenic composition in order to protect the antigen from rapid catabolism.

Many other types of materials can be used as adjuvants in immunogenic compositions according to the present invention. They include plant products such as saponin, animal products such as chitin and numerous synthetic chemicals.

Adjuvants according to the present invention can also been categorized by their proposed mechanisms of action. This type of classification is necessarily somewhat arbitrary because most adjuvants appear to function by more than one mechanism. Adjuvants may act through antigen localization and delivery, or by direct effects on cells making up the immune system, such as macrophages and lymphocytes. Another mechanism by which adjuvants according to the invention enhance the immune response is by creation of an antigen depot. This appears to contribute to the adjuvant activity of aluminum compounds, oil emulsions, liposomes, and synthetic polymers. The adjuvant activity of lipopolysaccharides and muramyl dipeptides appears to be mainly mediated through activation of the macrophage, whereas B. pertussis affects both macrophages and lymphocytes. Further examples of adjuvants that may be useful when incorporated into immunogenic compositions according to the present invention are described in US 5,554,372.

Adjuvants useful in compositions according to the present invention may thus be mineral salts, such as aluminium hydroxide and aluminium or calcium phosphates gels, oil emulsions and surfactant based formulations such as MF59 (microfluidized detergent stabilized oil in water emulsion), QS21 (purified saponin), AS02 (SBAS2, oil-in-water emulsion + monophosphoryl lipid A (MPL) + QS21), Montanide ISA 51 and ISA-720 (stabilized water in oil emulsion), Adjuvant 65 (containing peanut oil, mannide monooleate and aluminum monostearate), RIBI ImmunoChem Research Inc., Hamilton, Utah), particulate adjuvants, such as virosomes (unilamellar liposomal cehicles incorporating influenza haemagglutinin), AS04 (Al salt with MPL), ISCOMS (structured complex of saponins and lipids (such as cholesterol), polyactide co-glycolide (PLG), microbial derivatives (natural and synthetic) such as monophosphoryl lipid A (MPL), Detox (MPL + *M*. *Phlei* cell wall skeleton), AGP (RC-529 (synthetic acylated monosaccharide)), DC_choI (lipoidal immunostimulators able to self-organize into liposomes), OM-174 (lipid A derivative), CpG motifs (synthetic oligonucleotides containing immunostimulatory CpG motifs), modified bacterial toxins, LT and CT, with non-toxic adjuvant effects, Endogenous human immunomodulators, e.g., hGM-CSF or hIL-12 or Immudaptin (C3d tandem array), inert vehicles such as gold particles.

Additional examples of adjuvants comprise: Immunostimulatory oil emulsions (for example, water-in-oil, oil-in-water, water-in-oil-in-water such as e.g. Freund's incomplete adjuvant such as Montainde®, Specol, mineral salts such e.g. as Al(OH)₃, AlPO₄, microbial products, Saponins such as Qual A, synthetic products, as well as adjuvant formulations, and immune stimulatory complexes (ISCOMs) and cytokines, heat-inactivated bacteria/components, nanobeads, LPS, LTA. A list of other commonly used adjuvants is disclosed on pages 6-8 in WO 2003/089471, the list being hereby incorporated by reference.

Immunogenic compositions according to the invention may also contain diluents such as buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients. Neutral buffered saline or saline mixed with non-specific serum albumin are exemplary appropriate diluents.

Adjuvants are generally included in the immunogenic compositions in an amount according to the instructions of the manufacturer.

### Enhancing an immune response: Non-traditional adjuvants

### Cytokine modulation

For a vaccine to be effective, it must induce an appropriate immune response for a given pathogen. This can be accomplished by modifications to the form of antigen expressed (i.e. intracellular vs. secreted), the method and route of delivery, and the dose of DNA delivered. However, it can also be accomplished by the co-administration of plasmid DNA (pDNA) encoding immune regulatory molecules, e.g. cytokines, lymphokines or co-stimulatory molecules. These "genetic adjuvants", along with any of the 'traditional adjuvants' or 'other immunstimulatory adjuvants' as outlined herein, may be administered a number of ways:
- as a mixture of 2 separate plasmids, one encoding the immunogen and the other encoding the cytokine;
- as a single bi- or polycistronic vector, separated by spacer regions; or
- as a plasmid-encoded chimera, or fusion protein; or
- in its native form, i.e. a protein or nucleotide.

In general, co-administration of pro-inflammatory agents (such as various interleukins, tumor necrosis factor, and GM-CSF) plus TH2 inducing cytokines increase antibody responses, whereas pro-inflammatory agents and TH1 inducing cytokines decrease humoral responses and increase cytotoxic responses (which is more important in viral protection, for example). Co-stimulatory molecules like B7-1, B7-2 and CD40L are also sometimes used.

This concept has been successfully applied in topical administration of pDNA encoding IL-10. Plasmid encoded B7-1 (a ligand on APCs) has successfully enhanced the immune response in anti-tumor models, and mixing plasmids encoding GM-CSF and the circumsporozoite protein of P. yoelii (PyCSP) has enhanced protection against subsequent challenge (whereas plasmid-encoded PyCSP alone did not). GM-CSF may cause dendritic cells to present antigen more efficiently, and enhance IL-2 production and TH cell activation, thus driving the increased immune response. This can be further enhanced by first priming with a pPyCSP and pGM-CSF mixture, and later boosting with a recombinant poxvirus expressing PyCSP. However, co-injection of plasmids encoding GM-CSF (or IFN-γ, or IL-2) and a fusion protein of *P. chabaudi* merozoite surface protein 1 (C-terminus)-hepatitis B virus surface protein (PcMSP1-HBs) actually abolished protection against challenge, compared to protection acquired by delivery of pPcMSP1-HBs alone.

### Other immunstimulatory adjuvants

In one embodiment, any of the following may be used as an immunostimulatory adjuvant to the nucleic acid construct or composition according to the present invention:
LPS (lipopolysaccharide), Poly-IC (poly-inositol cytosine) or any other adjuvant that resembles double-stranded RNA, LL37, RIG-1 helicase, IL-12, IL-18, CCL-1, CCL-5, CCL-19, CCL-21, GM-CSF, CX3CL, CD86, PD-1, secreted PD-1, IL10-R, secreted IL10-R, IL21, ICOSL, 41BBL, CD40L and any other protein or nucleic acid sequence that stimulates an immune response.

In one embodiment, the immunostimulatory adjuvant is fused to an adenoviral fiber protein. For example, CX3CL may be fused to adenoviral fiber proteins.

### Immunostimulatory CpG motifs

Plasmid DNA itself appears to have an adjuvant effect on the immune system. Plasmid DNA has derived from bacteria been found to trigger innate immune defense mechanisms, the activation of dendritic cells, and the production of TH1 cytokines. This is due to recognition of certain CpG dinucleotide sequences which are immunostimulatory. CpG stimulatory (CpG-S) sequences occur twenty times more frequently in bacterially derived DNA than in eukaryotes. This is because eukaryotes exhibit "CpG suppression" - i.e. CpG dinucleotide pairs occur much less frequently than expected. Additionally, CpG-S sequences are hypomethylated. This occurs frequently in bacterial DNA, while CpG motifs occurring in eukaryotes are all methylated at the cytosine nucleotide. In contrast, nucleotide sequences which inhibit the activation of an immune response (termed CpG neutralising, or CpG-N) are over represented in eukaryotic genomes. The optimal immunostimulatory sequence has been found to be an unmethylated CpG dinucleotide flanked by two 5' purines and two 3' pyrimidines. Additionally, flanking regions outside this immunostimulatory hexamer are optionally guanine-rich to ensure binding and uptake into target cells.

The innate immune system works synergistically with the adaptive immune system to mount a response against the DNA encoded protein. CpG-S sequences induce polyclonal B-cell activation and the upregulation of cytokine expression and secretion. Stimulated macrophages secrete IL-12, IL-18, TNF-α, IFN-α, IFN-β and IFN-γ, while stimulated B-cells secrete IL-6 and some IL-12. Manipulation of CpG-S and CpG-N sequences in the plasmid backbone of DNA vaccines can ensure the success of the immune response to the encoded antigen, and drive the immune response toward a TH1 phenotype. This is useful if a pathogen requires a TH response for protection. CpG-S sequences have also been used as external adjuvants for both DNA and recombinant protein vaccination with variable success rates. Other organisms with hypomethylated CpG motifs have also demonstrated the stimulation of polyclonal B-cell expansion. However, the mechanism behind this may be more complicated than simple methylation - hypomethylated murine DNA has not been found to mount an immune response.

### Formulations of DNA

The efficiency of DNA immunization can be improved by stabilising DNA against degradation, and increasing the efficiency of delivery of DNA into antigen presenting cells. This may be achieved by coating biodegradable cationic microparticles (such as poly(lactide-co-glycolide) formulated with cetyltrimethylammonium bromide) with DNA. Such DNA-coated microparticles can be as effective at raising CTL as recombinant vaccinia viruses, especially when mixed with alum. Particles 300nm in diameter appear to be most efficient for uptake by antigen presenting cells.

### Administration

Nucleic acid constructs and compositions according to the invention may be administered to an individual in therapeutically effective amounts. The effective amount may vary according to a variety of factors such as the individual's condition, weight, sex and age. Other factors include the mode of administration.

In one embodiment, the nucleic acid construct according to the present invention may be delivered to a subject in the form of DNA, RNA, LNA, PNA, INA, TINA, HNA, ANA, CNA, CeNA, GNA, TNA, Gap-mers, Mix-mers, Morpholinos or any combination thereof.

In one embodiment, the nucleic acid construct according to the present invention may be delivered to a subject in the form of DNA.

In another embodiment, the nucleic acid construct according to the present invention may be delivered to a subject in the form of RNA. Thus, the nucleic acid construct may be transcribed into RNA prior to administration.

In yet another embodiment, the nucleic acid construct according to the present invention may be delivered to a subject in the form of protein. Thus, the nucleic acid construct may be translated into protein prior to administration.

In the embodiment in which the nucleic acid construct according to the present invention is delivered to a subject in the form of a protein, the protein may have been modified to increase stabilization and/or to optimize delivery into the cell. The protein may have increased stability due to the presence of disulfide bonds (for example, US 5,102,985 treated solutions of proteins in reduced form with hydrogen peroxide to generate proteins having an intramolecular disulfide bridge in 90-96% yield), an increase in polar residues, surface charge optimization, surface salt bridges, encapsulation (e.g. with mesoporous silicate), or the protein may be linked to heat-shock proteins (such as Hsp-60, Hsp-70, Hsp-90, Hsp-20, Hsp-27, Hsp-84 and others), HIV tat translocation domain, adenoviral fiber proteins, or any other proteins or domains.

The pharmaceutical or veterinary compositions may be provided to the individual by a variety of routes such as subcutaneous (sc or s.c.), topical, oral and intramuscular (im or i.m.). Administration of pharmaceutical compositions is accomplished orally or parenterally. Methods of parenteral delivery include topical, intra-arterial (directly to the tissue), intramuscular, intracerebrally (ic or i.c.), subcutaneous, intramedullary, intrathecal, intraventricular, intravenous (iv or i.v.), intraperitoneal, or intranasal administration. The present invention also has the objective of providing suitable topical, oral, systemic and parenteral pharmaceutical formulations for use in the methods of priming an immune response with the composition.

For example, the compositions can be administered in such oral dosage forms as tablets, capsules (each including timed release and sustained release formulations), pills, powders, granules, elixirs, tinctures, solutions, suspensions, syrups and emulsions, or by injection. Likewise, they may also be administered in intravenous (both bolus and infusion), intraperitoneal, subcutaneous, topical with or without occlusion, or intramuscular form, all using forms well known to those of ordinary skill in the pharmaceutical arts. An effective but non-toxic amount of the composition, comprising any of the herein described compounds can be employed. Also any and all conventional dosage forms that are known in the art to be appropriate for formulating injectable immunogenic peptide composition are encompassed, such as lyophilized forms and solutions, suspensions or emulsion forms containing, if required, conventional pharmaceutically acceptable carriers, diluents, preservatives, adjuvants, buffer components, etc.

In one embodiment, the composition for priming and/or the subsequent booster vaccine is given as a slow or sustained release formulation.

Preferred modes of administration of the nucleic acid construct or composition according to the invention include, but are not limited to systemic administration, such as intravenous or subcutaneous administration, intradermal administration, intramuscular administration, intranasal administration, oral administration, rectal administration, vaginal administration, pulmonary administration and generally any form of mucosal administration. Furthermore, it is within the scope of the present invention that the means for any of the administration forms mentioned in the herein are included in the present invention.

A nucleic acid construct or composition according to the present invention can be administered once, or any number of times such as two, three, four or five times.

In a preferred embodiment, the nucleic acid construct or composition is administered once, followed by administration of a suitable vaccine.

In another preferred embodiment, the nucleic acid construct or composition is administered as a series of administrations prior to administering the vaccine. Such a series may comprise administering the nucleic acid construct or composition daily, every second day, every third day, every fourth day, every fifth day, every sixth day, weekly, bi weekly or every third week for a total of one, two, three, four or five times.

In one embodiment, the time period between administering first the nucleic acid construct or composition for priming the immune system and secondly the vaccine for boosting is at least one day apart, such as at least two days apart, for example three days apart, such as at least four days apart, for example five days apart, such as at least six days apart, for example seven days apart, such as at least eight days apart, for example nine days apart, such as at least ten days apart, for example fifteen days apart, such as at least twenty days apart, for example twenty-five days apart.

Priming with the nucleic acid construct or composition is thus intended to be further boosted by administering a vaccine. Administration may be in a form or body part different from the previous administration or similar to the previous administration.

The booster shot is either a homologous or a heterologous booster shot. A homologous booster shot is a where the first and subsequent administrations comprise the same constructs and more specifically the same delivery vehicle. A heterologous booster shot is where identical constructs are comprised within different vectors.

A preferred administration form of the composition according to the present invention is administering the composition to the body area, inside or out, most likely to be the receptacle of a given infection. The receptacle of infection is the body area that the infection is received by, e.g. regarding influenza, the receptacle of infection is the lungs.

The nucleic acid construct or composition of the present invention can be administered to any organism to which it may be beneficial, especially any animal such as a vertebrate animal. It falls within the scope of the present invention that the means and modes of administration of the composition are adapted to the recipient.

A preferred recipient of the composition is a mammal and the mammal is in a more preferred embodiment of the present invention selected from the group of: cows, pigs, horses, sheep, goats, llamas, mice, rats, monkeys, dogs, cats, ferrets and humans. In the most preferred embodiment the mammal is a human.

Another preferred recipient of the composition is any vertebrate from the class aves (bird), such as Gallus gallus domesticus (chicken).

An embodiment of the present invention includes a composition further comprising a second active ingredient. The second active ingredient is selected from, but not limited the group of adjuvants, antibiotics, chemotherapeutics, anti-allergenics, cytokines, complement factors and co-stimulatory molecules of the immune system.

Another embodiment of the present invention comprises a kit of parts, wherein the kit includes at least one nucleic acid construct or composition according to any of the above, a means for administering said nucleic acid construct or composition and the instruction on how to do so. It is within the scope of the present invention to include multiple dosages of the same composition or several different compositions. In a preferred embodiment the kit of parts further comprises a second active ingredient. In a more preferred embodiment, said second active ingredient is a suitable vaccine, i.e. a vaccine capable of boosting the immune response raised by previous priming of said immune response.

The present invention further comprises a method for potentiating an immune response in an animal, comprising administering to the animal a nucleic acid construct or composition according to any of the above, followed by administering a suitable vaccine, thereby priming and boosting the immune system of a subject.

The immune response may be, but is not limited to, any of the following types of responses: an MHC-I dependent response, an MHC-I and/ or MHC-II dependent response, a T-cell dependent response, a CD4⁺ T-cell dependent response, a CD4⁺ T cell independent response, a CD8⁺ T-cell dependent response and a B cell dependent immune response. Suitable vaccines are those that are capable of boosting the immune system subsequent to the priming of the immune system with the nucleic acid construct or composition according to the present invention.

In a further embodiment, the present invention relates to a method of treatment of an individual in need thereof, comprising administering the composition as described herein above to treat a clinical condition in said individual.

### Increasing the potency of a vaccine

An embodiment of the invention relates to a nucleic acid construct encoding at least one invariant chain or variant thereof and at least one antigenic protein or peptide or fragment of an antigenic protein or peptide, wherein the at least one antigenic protein or peptide or fragment of an antigenic protein or peptide is from a virus, bacteria or parasite.

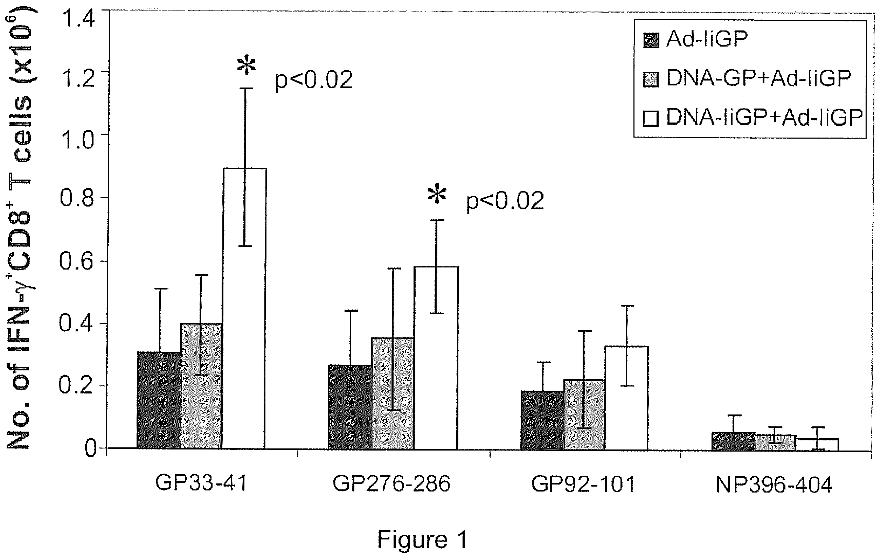

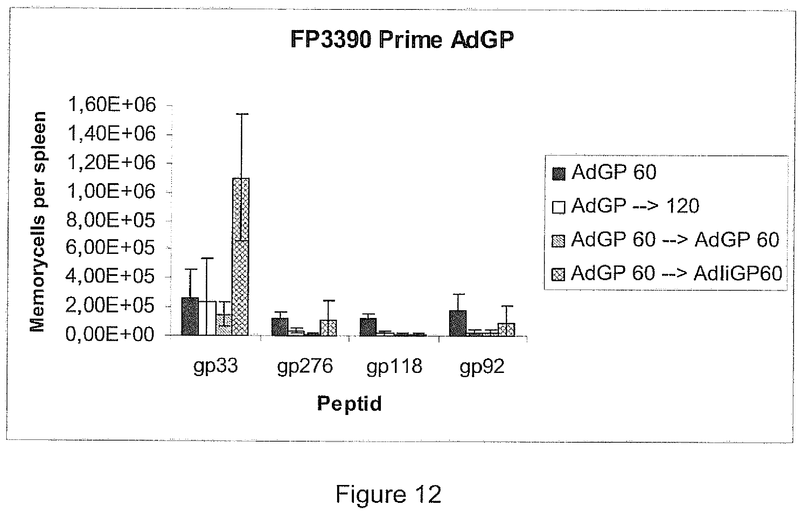

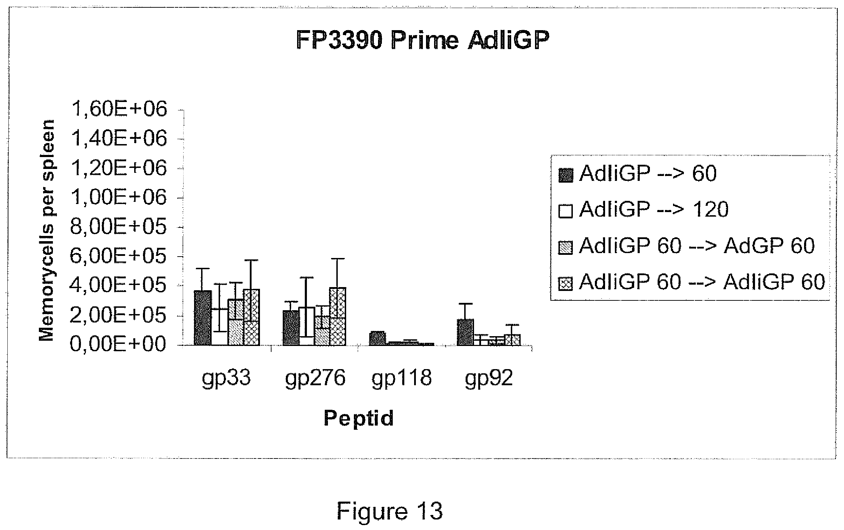

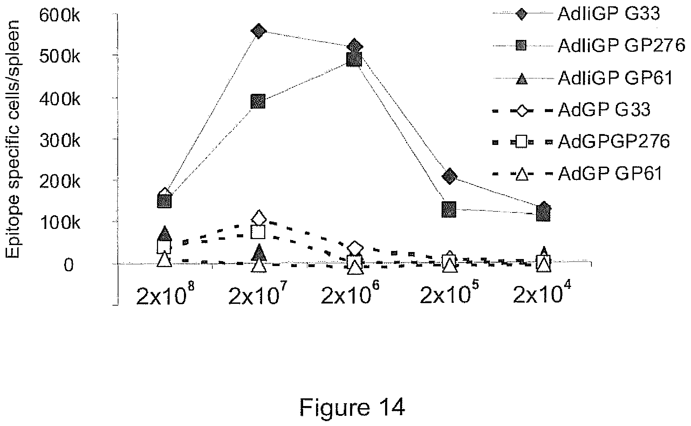

Data presented herein shows that it is not straightforward to develop prime-boost regimens using nucleic acid constructs comprising invariant chain or variant thereof. Thus, as presented in figure 1, a naked DNA construct comprising invariant chain and an antigen (DNA-liGP) is capable of priming an immune response, whereas a naked DNA construct comprising an antigen but not invariant chain (DNA-GP) is not capable of priming an immune response. Furthermore, data presented in figure 12 show that an adenoviral vector comprising an antigen (AdGP) is capable of priming certain (Ad-liGP) but not all (Ad-GP) immune responses, whereas data presented in figure 13 show that an adenoviral vector comprising invariant chain with an antigen (Ad-liGP) is not capable of priming any (Ad-liGP and Ad-GP) immune responses under normal dosage and treatment regimens. However, it is possible to optimize Ad-liGP priming of an Ad-liGP boost by using lower doses of Ad-liGP for priming (as shown in figure 14).

It is an object of the present invention to provide a nucleic acid construct encoding at least one invariant chain and a viral, bacterial or parasitic antigen or a fragment thereof, wherein said invariant chain is a variant of invariant chain, for priming an immune response, wherein said priming is followed by a subsequent booster vaccination with a cancer vaccine. Said variant of invariant chain may be any variant as specified elsewhere herein, comprising invariant chain wherein the li-KEY LRMK amino acid residues have been altered by e.g. deletion or substitution, or wherein part of the CLIP region has been altered by e.g. deletion or substitution.

In one embodiment, the present invention is directed to the use of a nucleic acid construct for increasing the potency of a vaccine.

In one embodiment, the present invention discloses a method for increasing the potency of a vaccine comprising the steps of:
a. providing a nucleic acid construct comprising invariant chain or a variant thereof and an antigenic peptide or fragment thereof,
b. priming the immune system of a subject by administering the nucleic acid construct of step a) thereby stimulating an immune response in said subject, and
c. boosting the immune response of step b) by administering a suitable vaccine.

In one embodiment, the present invention discloses a method for increasing the potency of a vaccine comprising the steps of:
a. providing a nucleic acid construct comprising a variant of invariant chain and an antigenic peptide or fragment thereof,
b. priming the immune system of a subject by administering the nucleic acid construct of step a) thereby stimulating an immune response in said subject, and
c. boosting the immune response of step b) by administering a suitable vaccine,
wherein said variant of invariant chain comprises alteration of the li-KEY LRMK amino acid residues by e.g. deletion or substitution.

In one embodiment, the present invention discloses a method for increasing the potency of a vaccine comprising the steps of:
a. providing a nucleic acid construct comprising a variant of invariant chain and an antigenic peptide or fragment thereof,
b. priming the immune system of a subject by administering the nucleic acid construct of step a) thereby stimulating an immune response in said subject, and
c. boosting the immune response of step b) by administering a suitable vaccine,
wherein said variant of invariant chain comprises alteration of the CLIP region by e.g. deletion or substitution.

In one embodiment, the present invention discloses a method for increasing the potency of a vaccine comprising the steps of:
a. providing a nucleic acid construct comprising a variant of invariant chain and an antigenic peptide or fragment thereof,
b. priming the immune system of a subject by administering the nucleic acid construct of step a) thereby stimulating an immune response in said subject, and
c. boosting the immune response of step b) by administering a suitable vaccine,
wherein said variant of invariant chain does not comprise the first 17 amino acids.

In another embodiment, the present invention is directed to the use of a nucleic acid construct for priming of an immune response.

In one embodiment, the present invention discloses a method for priming of an immune response comprising the steps of:
a. providing a nucleic acid construct comprising invariant chain or a variant thereof and an antigenic peptide or fragment thereof,
b. priming the immune system of a subject by administering the nucleic acid construct of step a) thereby stimulating an immune response in said subject, and
c. boosting the immune response of step b) by administering a suitable vaccine.

In one embodiment, the present invention discloses a method for priming of an immune response comprising the steps of:
a. providing a nucleic acid construct comprising a variant of invariant chain and an antigenic peptide or fragment thereof,
b. priming the immune system of a subject by administering the nucleic acid construct of step a) thereby stimulating an immune response in said subject, and
c. boosting the immune response of step b) by administering a suitable vaccine,
wherein said variant of invariant chain comprises alteration of the li-KEY LRMK amino acid residues by e.g. deletion or substitution.

In one embodiment, the present invention discloses a method for priming of an immune response comprising the steps of:
a. providing a nucleic acid construct comprising a variant of invariant chain and an antigenic peptide or fragment thereof,
b. priming the immune system of a subject by administering the nucleic acid construct of step a) thereby stimulating an immune response in said subject, and
c. boosting the immune response of step b) by administering a suitable vaccine,
wherein said variant of invariant chain comprises alteration of the CLIP region by e.g. deletion or substitution.

In one embodiment, the present invention discloses a method for priming of an immune response comprising the steps of:
a. providing a nucleic acid construct comprising a variant of invariant chain and an antigenic peptide or fragment thereof,
b. priming the immune system of a subject by administering the nucleic acid construct of step a) thereby stimulating an immune response in said subject, and
c. boosting the immune response of step b) by administering a suitable vaccine,
wherein said variant of invariant chain does not comprise the first 17 amino acids.

### Increasing the potency of a cancer vaccine

An embodiment of the invention relates to a nucleic acid construct encoding at least one invariant chain or variant thereof and at least one antigenic protein or peptide or fragment of an antigenic protein or peptide, wherein the at least one antigenic protein or peptide or fragment of an antigenic protein or peptide is from a cancer-specific polypeptide or cancer antigen.

It is an object of the present invention to provide a nucleic acid construct encoding at least one invariant chain and a cancer antigen or a fragment thereof, wherein said invariant chain is in its native, wild type form, for priming an immune response, wherein said priming is followed by a subsequent booster vaccination with a cancer vaccine.

It is also an object of the present invention to provide a nucleic acid construct encoding at least one invariant chain and a cancer antigen or a fragment thereof, wherein said invariant chain is a variant of invariant chain, for priming an immune response, wherein said priming is followed by a subsequent booster vaccination with a cancer vaccine. Said variant of invariant chain may be any variant as specified elsewhere herein, comprising invariant chain wherein the li-KEY LRMK amino acid residues have been altered by e.g. deletion or substitution, or wherein part of the CLIP region has been altered by e.g. deletion or substitution or wherein the first 17 amino acids have been deleted.

It follows that when the subsequently administered vaccine used for boosting an immune response is a cancer vaccine, the invariant chain encoded by the nucleic acid construct according to the present invention may be either in its native, wild type form, or it may be a variant of invariant chain.

In one embodiment, the present invention is directed to the use of a nucleic acid construct for increasing the potency of a cancer vaccine.

In one embodiment, the present invention discloses a method for increasing the potency of a cancer vaccine comprising the steps of:
a. providing a nucleic acid construct comprising invariant chain or a variant thereof and an cancer-specific antigenic peptide or fragment thereof,
b. priming the immune system of a subject by administering the nucleic acid construct of step a) thereby stimulating an immune response in said subject, and
c. boosting the immune response of step b) by administering a suitable cancer vaccine.

In one embodiment, the present invention discloses a method for increasing the potency of a cancer vaccine comprising the steps of:
a. providing a nucleic acid construct comprising invariant chain and an cancer-specific antigenic peptide or fragment thereof,
b. priming the immune system of a subject by administering the nucleic acid construct of step a) thereby stimulating an immune response in said subject, and
c. boosting the immune response of step b) by administering a suitable cancer vaccine,
wherein said invariant chain is in its native, wild type form.

In one embodiment, the present invention discloses a method for increasing the potency of a cancer vaccine comprising the steps of:
a. providing a nucleic acid construct comprising a variant of invariant chain and an cancer-specific antigenic peptide or fragment thereof,
b. priming the immune system of a subject by administering the nucleic acid construct of step a) thereby stimulating an immune response in said subject, and
c. boosting the immune response of step b) by administering a suitable cancer vaccine,
wherein said variant of invariant chain comprises alteration of the li-KEY LRMK amino acid residues by e.g. deletion or substitution and/or alteration of part of the CLIP region by e.g. deletion or substitution, and/or deletion of the first 17 amino acids of li.

In another embodiment, the present invention is directed to the use of a nucleic acid construct for priming of an immune response.

In one embodiment, the present invention discloses a method for priming of an immune response comprising the steps of:
a. providing a nucleic acid construct comprising invariant chain or a variant thereof and an cancer-specific antigenic peptide or fragment thereof,
b. priming the immune system of a subject by administering the nucleic acid construct of step a) thereby stimulating an immune response in said subject, and
c. boosting the immune response of step b) by administering a suitable cancer vaccine.

In one embodiment, the present invention discloses a method for priming of an immune response comprising the steps of:
a. providing a nucleic acid construct comprising invariant chain and an cancer-specific antigenic peptide or fragment thereof,
b. priming the immune system of a subject by administering the nucleic acid construct of step a) thereby stimulating an immune response in said subject, and
c. boosting the immune response of step b) by administering a suitable cancer vaccine,
wherein said invariant chain is in its native, wild type form.

In one embodiment, the present invention discloses a method for priming of an immune response comprising the steps of:
a. providing a nucleic acid construct comprising a variant of invariant chain and an cancer-specific antigenic peptide or fragment thereof,
b. priming the immune system of a subject by administering the nucleic acid construct of step a) thereby stimulating an immune response in said subject, and
c. boosting the immune response of step b) by administering a suitable cancer vaccine,
wherein said variant of invariant chain comprises alteration of the li-KEY LRMK amino acid residues by e.g. deletion or substitution and/or alteration of part of the CLIP region by e.g. deletion or substitution, and/or deletion of the first 17 amino acids of li.

### Increasing the potency of a vaccine directed at an abnormal physiological response

It is an object of the present invention to provide a nucleic acid construct encoding at least one invariant chain and a polypeptide associated with an abnormal physiological response or a fragment thereof, wherein said invariant chain is in its native, wild type form, for priming an immune response, wherein said priming is followed by a subsequent booster vaccination with a vaccine directed at said abnormal physiological response.

It is also an object of the present invention to provide a nucleic acid construct encoding at least one invariant chain and a polypeptide associated with an abnormal physiological response or a fragment thereof, wherein said invariant chain is a variant of invariant chain, for priming an immune response, wherein said priming is followed by a subsequent booster vaccination with a vaccine directed at said abnormal physiological response. Said variant of invariant chain may be any variant as specified elsewhere herein, comprising invariant chain wherein the li-KEY LRMK amino acid residues have been altered by e.g. deletion or substitution, or wherein part of the CLIP region has been altered by e.g. deletion or substitution, or wherein the first 17 amino acids of li have been deleted.

It follows that when the subsequently administered vaccine used for boosting an immune response is a vaccine directed at said abnormal physiological response, the invariant chain encoded by the nucleic acid construct according to the present invention may be either in its native, wild type form, or it may be a variant of invariant chain.

In one embodiment, the present invention is directed to the use of a nucleic acid construct for increasing the potency of a vaccine directed at an abnormal physiological response.

In another embodiment, the present invention is directed to the use of a nucleic acid construct for priming of an immune response.

In one embodiment, the present invention discloses a method for increasing the potency of a vaccine directed at an abnormal physiological response comprising the steps of:
a. providing a nucleic acid construct comprising invariant chain or a variant thereof and an antigenic peptide or fragment thereof associated with an abnormal physiological response,
b. priming the immune system of a subject by administering the nucleic acid construct of step a) thereby stimulating an immune response in said subject, and
c. boosting the immune response of step b) by administering a suitable vaccine directed at an abnormal physiological response.

In one embodiment, the present invention discloses a method for increasing the potency of a vaccine directed at an abnormal physiological response comprising the steps of:
a. providing a nucleic acid construct comprising invariant chain and an antigenic peptide or fragment thereof associated with an abnormal physiological response,
b. priming the immune system of a subject by administering the nucleic acid construct of step a) thereby stimulating an immune response in said subject, and
c. boosting the immune response of step b) by administering a suitable vaccine directed at an abnormal physiological response,
wherein said invariant chain is in its native, wild type form.

In one embodiment, the present invention discloses a method for increasing the potency of a vaccine directed at an abnormal physiological response comprising the steps of:
a. providing a nucleic acid construct comprising a variant of invariant chain and an antigenic peptide or fragment thereof associated with an abnormal physiological response,
b. priming the immune system of a subject by administering the nucleic acid construct of step a) thereby stimulating an immune response in said subject, and
c. boosting the immune response of step b) by administering a suitable vaccine directed at an abnormal physiological response,
wherein said variant of invariant chain comprises alteration of the li-KEY LRMK amino acid residues by e.g. deletion or substitution and/or alteration of part of the CLIP region by e.g. deletion or substitution, and/or deletion of the first 17 amino acids of li.

### Vaccine types

One aspect of the present invention relates to the priming of an immune response in a subject by administering a nucleic acid construct comprising li-linked antigen, followed by a subsequent booster achieved by administering to the same subject a suitable vaccine.

Suitable vaccines according to the present invention have at least one identical feature in common with the nucleic acid construct used for priming of an immune response. Said identical feature may be comprised in part or all of an invariant chain, part or all of an antigenic peptide, part or all of a backbone structure such as part or all of a promoter region, part or all of an enhancer, part or all of a terminator, part or all of a poly-A tail, part or all of a linker, part or all of a polylinker, part or all of an operative linker, part or all of a multiple cloning site (MCS), part or all of a marker, part or all of a STOP codon, part or all of an internal ribosomal entry site (IRES) and part or all of a host homologous sequence for integration or other defined elements.

In a preferred embodiment, the identical feature is part or all of an antigenic peptide or a ubiquitous helper T cell epitope. In a most preferred embodiment, the identical feature is part or all of an antigenic peptide.

In another preferred embodiment, the identical feature is part or all of invariant chain.

Vaccines may be regarded as traditional or innovative. Any of the herein cited types of vaccines may be used in the subsequent booster step according to the present invention.

Traditional vaccines, or first generation vaccines, rely on whole organisms; either pathogenic strains that have been killed, or strains with attenuated pathogenicity.

Molecular biological techniques have been used to develop new vaccines, second generation vaccines, based on individual antigenic proteins from the pathogenic organisms. Conceptually, use of antigenic peptides rather than whole organisms would avoid pathogenicity while providing a vaccine containing the most immunogenic antigens. These include toxoid-based vaccines based on inactivated toxic compound are well-known, and subunit vaccines based on a fragment of an inactivated or attenuated pathogenic strain.

Conjugate vaccines: Certain bacteria have polysaccharide outer coats that are poorly immunogenic. By linking these outer coats to proteins (e.g. toxins), the immune system can be led to recognize the polysaccharide as if it was a protein antigen.

Recombinant vector vaccine: By combining the physiology of one micro-organism and the DNA of the other, immunity can be created against diseases that have complex infection processes.

Synthetic vaccines are composed mainly or wholly of synthetic peptides, carbohydrates or antigens.

DNA (or genetic) vaccines, or third generation vaccines, are new and promising candidates for the development of both prophylactic and therapeutic vaccines. DNA vaccines are made up of a small, circular piece of DNA (a plasmid) that has been genetically engineered to produce one or more antigens from a micro-organism. The vaccine DNA is injected into the cells of the body, where the "inner machinery" of the host cells "reads" the DNA and converts it into pathogenic proteins. Because these proteins are recognised as foreign, they are processed by the host cells and displayed on their surface, to alert the immune system, which then triggers a range of immune responses. The strength of the ensuing immune response is determined through a combination of the potency of the vector (i.e. naked DNA, viral vectors, live attenuated viruses etc.), the expression level of the antigen, and the recombinant antigen it self (i.e. high or low affinity MHC binders, structural determinants selecting for more or less limited T-or B-cell repertoire etc.). It is generally held to be true, that efficient induction of immunological memory requires or benefits from the interactions of CD4⁺ (helper cell) T-cells with CD8⁺ (cytotoxic) T-cells and B-cells that mediate many of the effects of immune memory.

In one embodiment of the present invention, priming of an immune response with a nucleic acid construct according to the present invention is followed by the subsequent administration of a first generation or traditional vaccine for boosting said immune response.

In one embodiment of the present invention, priming of an immune response with a nucleic acid construct according to the present invention is followed by the subsequent administration of a second generation vaccine for boosting said immune response.

In one embodiment of the present invention, priming of an immune response with a nucleic acid construct according to the present invention is followed by the subsequent administration of a third generation or DNA vaccine for boosting said immune response.

The use of invariant chain in DNA vaccine constructs to increase immunogenicity is well-known in the art. In one embodiment of the present invention, priming of an immune response with a nucleic acid construct according to the present invention is followed by the subsequent administration of a DNA vaccine comprising invariant chain or a variant thereof for boosting said immune response.

In one embodiment of the present invention, priming of an immune response with a nucleic acid construct according to the present invention is followed by the subsequent administration of an adenoviral vaccine for boosting said immune response.

Vaccines may further be *monovalent* (also called *univalent*) or *multivalent* (also called *polyvalent*). A monovalent vaccine is designed to immunize against a single antigen or single microorganism. A multivalent or polyvalent vaccine is designed to immunize against two or more strains of the same microorganism, or against two or more microorganisms.

### Detailed description of the drawings

**Figure 1****: DNA-priming with an li chain based naked DNA vaccine significantly augments the generation of virus-specific CD8⁺T cells upon subsequent boosting with a highly efficient viral vector.** Mice were gene-gun immunized twice 3 weeks apart with DNA-liGP, DNA-GP or left untreated. Three weeks after last immunization, all the mice were injected in the right hind footpad with 2x10⁷ IFU Ad5-liGP, and 4 weeks later the animals were sacrificed, and splenocytes were analyzed as described in Fig.1. Numbers of epitope-specific IFN-γ⁺CD8⁺ T cells are presented as mean ± SE (n=5 mice/group). * denotes statistical significance relative to mice vaccinated with Ad5-liGP only (Mann-Whitney rank-sum test). Results from one of two similar experiments are depicted.

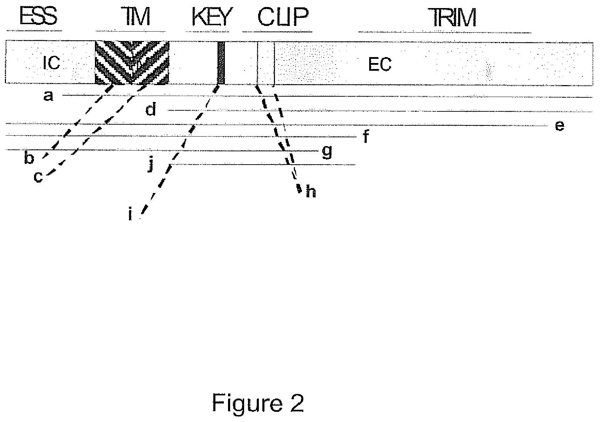

**Figure 2****: Location of the domains and the tested mutations in the li sequence.** Domains in WT li are depicted above the bar. ESS; endosomal sorting signal, TM; transmembrane domain, KEY; peptide presentation enhancing region, CLIP; class-II-associated invariant chain peptide, TRIM; trimerization domain. Extent of deletion mutations and substitutions in li is marked below the bar. A; Ad-Δ17liGP, b; Ad-liLTMGP, c; Ad-liUTMGP, d; Ad-Δ50liGP, e; Ad-li1-201GP, f; Ad-li1-118GP, g; Ad-li1-105GP, h; Ad-liCLIPGP, i; Ad-liKEYGP, j; Ad-li51-118GP.

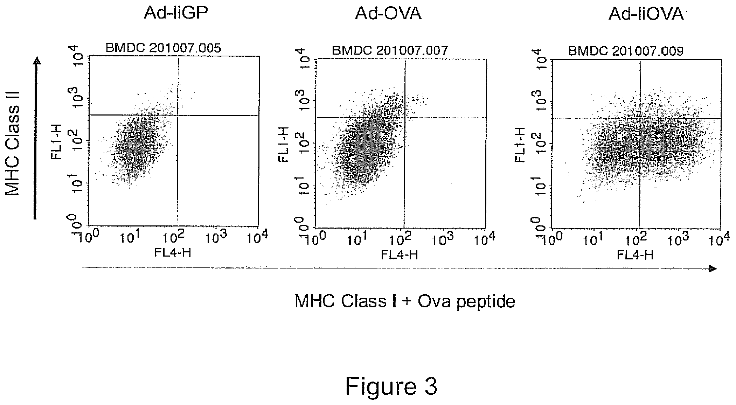

**Figure 3****: li dramatically increases cell surface presentation of the SIINFEKL/H-2kb OVA derived epitope.** Bone Marrow derived Dendritic Cells were transfected with Ad-OVA, Ad-liOVA or Ad-liGP (negative control), and surface stained for MHC class II (stains mature dendritic cells) and with a SIINFEKL/H-2kb specific antibody (OVA epitope).

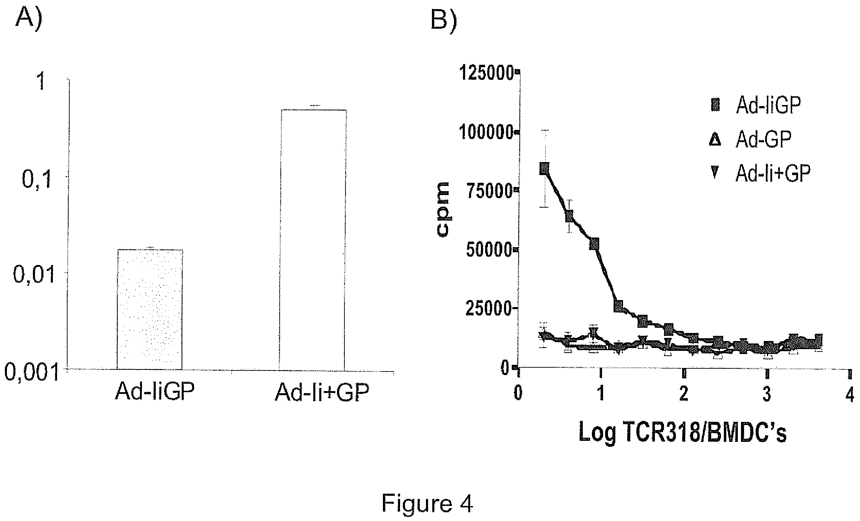

**Figure 4****: li works only in cis.** A) Expression of li from Ad-liGP and Ad-li + GP vectors; li expression was normalised to GAPDH in COS7 cells infected with 50 moi of Ad-liGP and Ad-li + GP. B) TCR318 GP33 restricted T-cell proliferation in response to Ad-GP, Ad-liGP or Ad-li + GP transduced BMDCs (bone marrow derived dendritic cell).

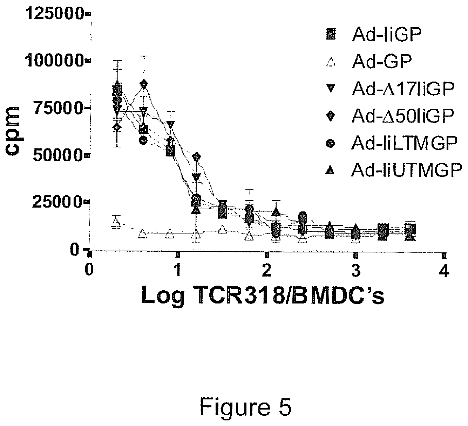

**Figure 5****: N-terminal deletions and substitutions does not effect li stimulatory capacity.** TCR 318 GP33 restricted T-cells proliferation in response to Ad-GP, Ad-liGP, Ad-Δ17liGP, Ad-liLTMGP, Ad-liUTMGP, Ad-Δ50liGP transduced BMDCs (bone marrow derived dendritic cell).

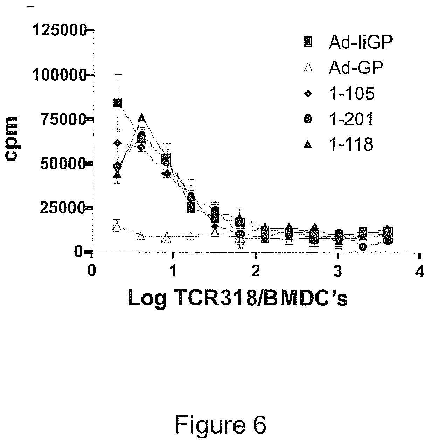

**Figure 6****: C-terminal deletions and substitutions does not effect li stimulatory capacity.** TCR 318 GP33 restricted T-cells proliferation in response to Ad-GP, Ad-liGP, Ad-li1-205GP, Adli1-118GP and Ad-li1-105GP transduced BMDCs (bone marrow derived dendritic cell culture system).

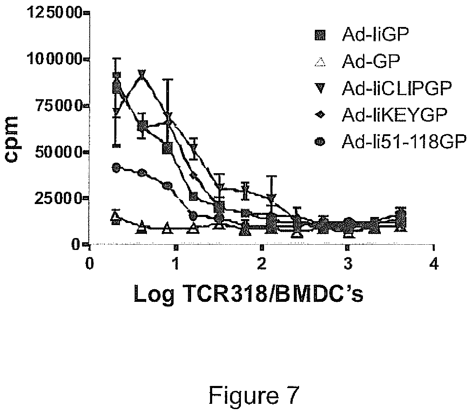

**Figure 7****: Only a N- and C-terminal deletion reduces li stimulatory capacity.** TCR 318 GP33 restricted T-cells proliferation in response to Ad-GP, Ad-liGP, Ad-liCLIPGP, Ad-liKEYGP and Ad-li51-118GP transduced BMDCs (bone marrow derived dendritic cell culture system).

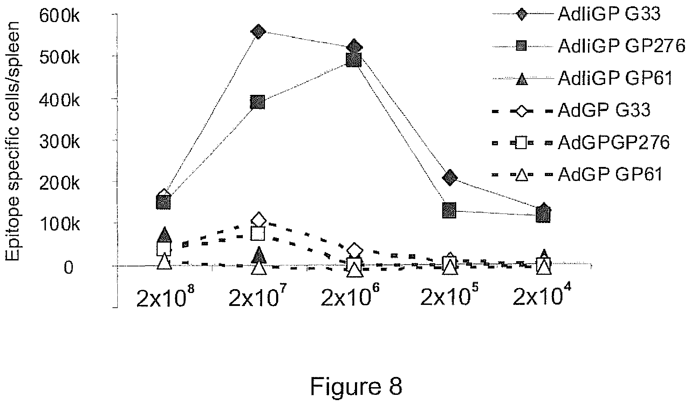

**Figure 8****: Dose-response of Ad-liGP and Ad-GP vaccines.** Groups of mice were vaccinated with the indicated vaccines in the indicated strains. 14 days after vaccination mice were sacrificed, and splenocytes stimulated with the indicated epitopes. Total number of specific CD8+ splenocytes was determined by intracellular staining and FACS analysis. The data shows that Ad-liGP induces responses at very low dosages, and thus priming with a low dose Ad-liGP (or any antigen) and subsequent boosting with a higher dose Ad-liGP (or any antigen) may be applicable for homologous prime-boost regimens.

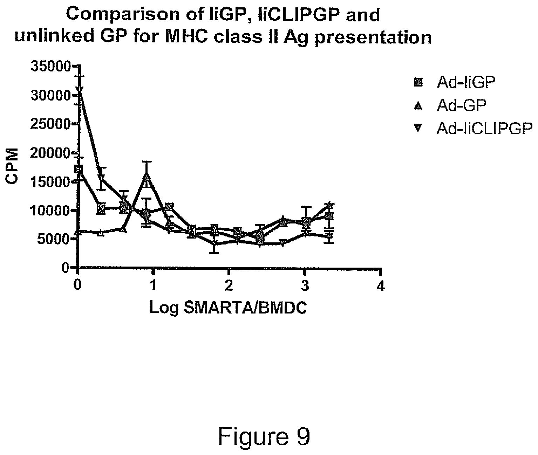

**Figure 9****: Comparison of Ad-GP, Ad-liGP and Ad-liCLIPGP for MHC class II presentation** (stimulation of CD4+ T-cells). SMARTA GP61-80 restricted T-cells proliferation in response to Ad-GP, Ad-liGP and Ad-liCLIPGP transduced BMDC's show an increased MHCII antigen presentation of Ad-liCLIPGP.

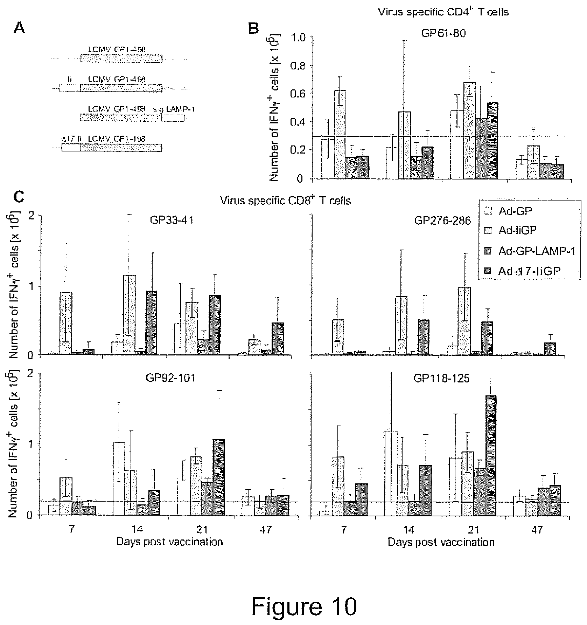

**Figure 10****: Comparison of Ad-GP, Ad-liGP, Ad-GPLamp-1 and Ad-liΔI7GP in an in vivo time-course study.**

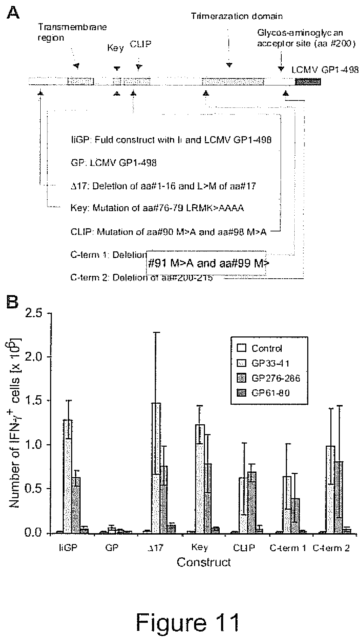

**Figure 11****: Comparison of Ad-GP, Ad-liGP, Ad-liΔ17GP, Ad-liKEYGP, Ad-liCLIPGP, Ad-li1-117GP and Ad-li1-199GP in vivo responses.**

**Figure 12****: Ad-GP is capable of priming a subsequent Ad-liGP boost.** 3 Groups of C57BL/6 mice were vaccinated with Ad-GP. 60 days later these mice were either left undisturbed, vaccinated with Ad-GP or vaccinated with Ad-liGP. A 4^{th} group of mice were included which were vaccinated with Ad-GP. 120 days after the first vaccinations, mice were sacrificed and antigen specific cells recognizing the indicated epitopes where quantitated by ex vivo restimulation with said peptides and intracellular staining for interferon-y production.

**Figure 13****: Ad-liGP is not capable of priming a subsequent Ad-GP or Ad-liGP boost.** 3 Groups of C57BL/6 mice were vaccinated with Ad-liGP. 60 days later these mice were either left undisturbed, vaccinated with Ad-GP or vaccinated with Ad-liGP. A 4^{th} group of mice were included which were vaccinated with Ad-liGP. 120 days after the first vaccinations, mice were sacrificed and antigen specific cells recognizing the indicated epitopes where quantitated by ex vivo restimulation with said peptides and intracellular staining for interferon-y production. This shows that Ad-liGP priming can not be boosted with Ad-liGP, whereas DNA-liGP priming can be boosted with Ad-liGP (see figure 1).

**Figure 14****: Dose-response of Ad-GP and Adli-Gp vaccines.** Groups of mice were vaccinated with the indicated vaccine in the indicated strains. 14 days after vaccination mice were sacrificed, and splenocytes stimulated with the indicated epitopes. Total number of CD8+ splenocytes was determined by intracellular staining and FACS analysis.

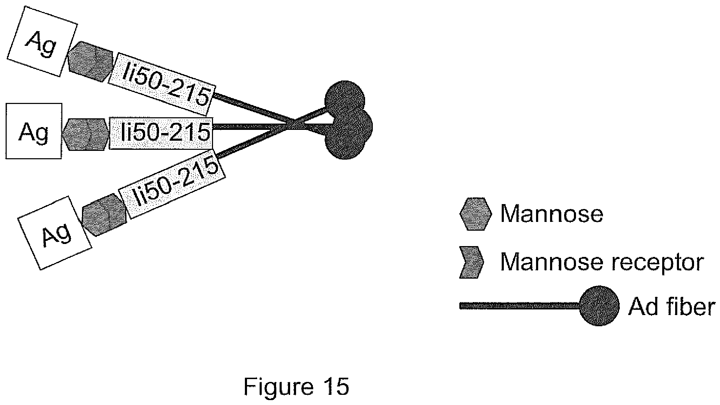

**Figure 15****: The Mannose receptor coupled to a variant of invariant chain comprising residues 50 to 215 (li50-215), further coupled to an adenoviral fiber protein.** The adenoviral fiber protein (Ad fiber) may stem from any serotype of adenovirus. The mannose receptor may be one or more domains from the Mannose receptor. The li may be a variant of or full length li. Ag = Antigen.

### Examples of the Invention

The invention will now be further illustrated with reference to the following examples. It will be appreciated that what follows is by way of example only and that modifications in detail may be made while still falling within the scope of the invention.

### Example 1: Priming with an li chain based naked DNA vaccine significantly augments the generation of virus-specific CD8⁺T cells upon subsequent boosting with an optimized viral vector.

Priming with a naked DNA vaccine (i.e. a nucleic acid construct) is shown to augment the immune response raised by subsequent immunization with Ad5 (adenovirus serotype 5) vector. Priming with DNA-liGP (DNA construct expressing LCMV (lymphocytic choriomeningitis virus) glycoprotein (GP) fused to invariant chain (li)) is herein demonstrated to significantly enhance the CD8⁺ T-cell response induced by the same gene construct delivered in an adenovirus serotype 5 vector (Ad5-liGP), providing a strong argument for the inclusion of li chain based DNA-constructs in future heterologous immunization ("prime-boost") protocols.

Our study shows that the immunoenhancing effect of li chain linkage is not limited to the Ad5 vector, but is relevant on a DNA platform as well. Furthermore, given the fact that li chain enhances presentation of more than one epitope, this places li chain based DNA vaccines as very promising candidates for various heterologous prime-boost regimes.

### Results & discussion

One way to improve the induced T-cell memory is through heterologous prime-boost regime e.g. naked DNA priming followed by a vector boost. Thus having in our laboratory the appropriate vector, replication deficient adenovirus expressing LCMV GP fused to p31 li chain (Ad5-liGP) this possibility was tested experimentally. First, we performed standard DNA vaccination, gene-gun-vaccination twice 3 weeks apart with DNA-liGP or DNA-GP. Three weeks after the second DNA-vaccination, both groups of mice and matched controls were immunized by inoculation of 2x10⁷ IFU Ad5-liGP in the right hind footpad, and 4 weeks later the number of virus-specific CD8⁺ T cells in the spleen was enumerated by way of ICCS for IFN-γ and flow cytometry. Mice primed with the fused DNA construct contained significantly more GP₃₃₋₄₁ and GP₂₇₆₋₂₈₆-specific IFN-γ⁺CD8⁺T cells than did unprimed mice, and a similar trend was noted for GP₉₂₋₁₀₁-specific cells, although in this case the difference was not statistically significant. In contrast, priming with naked DNA encoding GP in the absence of li had little effect on the level of GP-specific memory CD8⁺ T cells induced by subsequent immunization with Ad5-liGP (figure 1). It should be noted that the observed effect of including li does not reflect non-specific augmentation of the immunoreactivity of vaccinated mice, as DNA priming with a vector including only li, but no GP, had no effect on the level of GP-specific CD8⁺ T cells in mice subsequently inoculated with the adenoviral vector (data not shown).

We have shown that use of the improved DNA-vector as a part of a heterologous prime-boost regime will significantly augment the response induced by an already optimized viral vector (Holst *et al*., 2008). This strongly indicates that even very immunogenic vector based immunization may be further improved through initial priming of the host with an li chain based naked DNA vaccine. Altogether, since li chain fusion to the antigen will lead to priming for a broad CD8⁺ T cell response, li chain based DNA vaccines should represent a clear advantage with regard to prevention strategies against rapidly mutating viruses as part of heterologous prime-boost regimes.

### Materials and methods

*Mice.* C57BL/6 (B6) wild type mice were obtained from Taconic M&B (Ry, Denmark). Perforin deficient B6 mice were bred locally from breeder pairs originally obtained from The Jackson Laboratory (Bar Harbor, ME). Seven- to 10-week-old mice were used in all experiments, and animals from outside sources were always allowed to acclimatize to the local environment for at least 1 week before use. All animals were housed under specific pathogen free conditions as validated by screening of sentinels. All animal experiments were conducted according to national guidelines.

*DNA vaccine construction and immunization procedure.* The DNA vaccines are produced using the eukaryotic expression vector pACCMV.pLpA containing either the murine invariant chain followed by GP of LCMV or LCMV GP alone. The constructs were generated as recently described (Holst *et al*., 2008). The E. coli strain XL1-blue (Stratagene, USA) was transformed with the constructs by electroporation. DNA sequencing using cycle sequencing, Big Dye Terminator and ABI310 genetic analyzer (ABIprism, USA) identified positive clones. Primers were obtained from TAG, Copenhagen, Denmark. Large scale DNA preparations were produced using Qiagen Maxi Prep (Qiagen, USA).

*Gene-gun immunization.* DNA was coated onto 1.6 nm gold particles in a concentration of 2 µg DNA/mg gold, and the DNA/gold complexes were coated onto plastic tubes such that 0.5 mg gold was delivered to the mouse pr. shot (1 µg DNA pr. shot). These procedures were performed according to the manufacturer's instructions (Biorad, CA, USA) (Bartholdy *et al*., 2003). Mice were immunized on the abdominal skin using a hand held gene-gun device employing compressed Helium (400 psi) as the particle motive force. Unless otherwise mentioned, mice were immunized twice with an interval of 3-4 weeks and then allowed to rest for 3 weeks before further challenge/ investigation.

*Virus.* LCMV of the Armstrong strain clone 13 was used in most experiments. Unless otherwise stated, mice to be infected received a dose of 10⁵ pfu of clone 13 in an i.v. injection of 0.3 ml, or 20 pfu in 0.03 ml in the right hind footpad (f.p.). For i.c. injection mice received 20 pfu of neurotropic Armstrong clone 53b in a volume of 0.03 ml. Replication deficient adenovirus encoding invariant chain linked GP (Ad5-liGP) was produced and titrated as recently described (Holst *et al*., 2008).

*Virus titration.* Organ virus titers were assayed by an immune focus assay as previously described (Battegay *et al*., 1991).

*In vivo depletion of CD4⁺ and CD8⁺ T cells.* The anti-CD4 (clone GK1.5) and anti-CD8 mAbs (clone 53.6.72) were used. Mice to be depleted of cells received a dose of 200 □g in a volume of 0.3 ml PBS intraperitoneally on days -1 and 0 relative to infection; for sham treatment purified rat IgG (Jackson ImmunoResearch) was used instead. The efficiency of cell depletion was verified by flow cytometry.

*Survival study.* Mortality was used to evaluate the clinical severity of acute LCMV induced meningitis. Mice were checked twice daily for a period of 14 days or until 100% mortality was reached.

*Assay of LCMV-specific footpad swelling reaction.* Mice were infected locally in the right hind footpad as described above, and the local swelling reaction was followed until day 14 p.i. Footpad thickness was measured with a dial caliper (Mitutoyo 7309, Mitutoyo Co., Tokyo, Japan), and virus-specific swelling was determined as the difference in thickness of the infected right and the uninfected left foot (Christensen *et al*., 1994).

*Cell preparations.* Spleens from mice were aseptically removed and transferred to Hanks' balanced salt solution (HBSS). Single cell suspensions were obtained by pressing the organs through a fine sterile steel mesh. The cells were washed twice with HBSS, and cell concentration was adjusted in RPMI 1640 containing 10% fetal calf serum (FCS), supplemented with 2-mercaptoethanol, L-glutamin, and penicillin-streptomycin solution.

*mAb for flow cytometry.* The following mAbs were all purchased from PharMingen (San Diego, CA) as rat anti-mouse antibodies: FITC-conjugated anti-CD44, Cy-Chrome conjugated anti-CD8a, Cy-Chrome conjugated anti-CD4 and Phycoerythrin(PE)-conjugated anti IFN-γ̃.

*Flow cytometric analysis.* For visualization of LCMV-specific (interferon-y producing) CD8⁺/CD4⁺ T cells, 1-2 x 10⁶ splenocytes were resuspended in 0.2 ml complete RPMI medium supplemented with 10 units murine recombinant IL-2 (R&D Systems Europe Ltd, Abingdon, UK), 3 µM monensin (Sigma Chemicals co., St Louis, MO) and 1µg/ml relevant peptide and incubated for 5 hours at 37 º C. The following peptides were used: for CD8⁺ T cells GP33-41, GP276-86, GP92-101, GP118-125, and NP396-404 for control; for CD4⁺ T cells GP61-80. After incubation, cells were surface stained, washed, permeabilized and stained with IFN-γ specific mAb as described previously (Andreasen *et al*., 2000; Christensen *et al*., 2003). Isotype matched antibody served as control for non-specific staining. Cells were analyzed using a FACS Calibur (Becton Dickinson, San Jose, CA), and at least 10⁴ live cells were gated using a combination of low angle and side scatter to exclude dead cells and debris. Data analysis was conducted using Cell-Quest software.

### Example 2: Enhanced CD8+ T-cell activation of li linked antigen is independent of native li

The li sequence contains multiple regions with functions in antigen processing including: a cytoplasmic sorting domain and trimerization domain, a cytoplasmic and proximal membrane signalling domain, cytoplasic, intramembrane and periplasmic trimerization domains , the "key" motif involved in unlocking MHC molecules to facilitate binding of exogenous peptides, binding motifs for MHC class I and II in the CLIP region, a periplasmic glycosylation site as well as a structurally unidentified region of interaction with CD44 and Macrophage migration Inhibitory Factor (MIF) (figure 2).

li linkage increases the antigen presentation on both MHC class I and II. By using Ad-liOVA (OVA is ovalbumin) or Ad-OVA transduction of Bone Marrow derived Dendritic Cells (BMDC), we found that li linkage did indeed induce a dramatic increase in MHC class I restricted antigen presentation, as measured by direct staining with an antibody directed against the SIINFEKL OVA epitope presented on H-2Kb (figure 3). The increased MHC class I restricted antigen expression from the li linked sequences works directly on the APC independently of MHC class II, CD4+ T cells, and any other cell type and can be directly measured in dendritic cell cultures.

### li in cis

To establish whether li works only *in cis* or also *in trans,* an additional reading frame into the adenoviral vector was established by synthesizing a phosphoglycerate kinase (pGK) promoter with a β-globin polyadenylation signal and cloning this into the E3 region of the adenoviral backbone. This vector could then be used for recombination with the shuttle vector used to create the Ad-GP vector (which expresses LCMV GP from the E1 reading frame under control of the human CMV promoter and SV40 polyA). The new vector expresses LCMV GP from the adenoviral E1 region and li from the E3 region (Ad-li+GP). The promoter was verified for the induction of green fluorescent cells by transfection into COS7 cells, and a measurement of li mRNA expression in Ad-liGP and Ad-li+GP infected COS7 cells confirms that li is at least as efficiently expressed from the pGK promoter as from the CMV promoter (figure 4A). Comparing of TCR318 cells stimulated with Ad-GP, Ad-liGP and Ad-li+GP infected BMDC's clearly show that li must be linked to the antigen to have any effect (figure 4B). It would have been surprising if li expression *in trans* had shown efficacy as the BMDC cultures used for the stimulation already express li.

### N-terminal alterations:

Starting from the N-terminal, we made 1) a deletion of the first 17 amino acids (Ad-Δ17liGP), which removes the Leucine based endosomal sorting signals, 2) a replacement of the first half of the transmembrane segment (Ad-liLTMGP), with the corresponding segment from the chemokine receptor CCR6 TM6, 3) a replacement of the second half of the transmembrane segment with the corresponding CCR6 TM6 segment (Ad-liUTMGP), and finally 4) a complete deletion of the first 50 amino acids (Ad-Δ50liGP). The latter deletion removed the entire cytosolic, TM and membrane proximal region. None of these mutations had any effect on the ability of the remaining li sequence to enhance stimulation of CD8+ T cells (figure 5).

### C-terminal alterations:

From the C-terminus we made deletions of the last 14 aa (Ad-li1-201GP, this removes the C-terminal glycosylation signal), the last 97 aa (Ad-li1-118GP), and the last 110 aa (Ad-li1-105GP). No effect on the ability of the remaining li sequence to enhance stimulation of CD8+ T cells was observed by the 1-201, whereas only inconsistent and minor trends of reductions could be seen from the 1-105 mutation and the 1-118 mutations (figure 6).

### Mutations:

We also attempted to make point mutations in the reported MHC class I binding site of the CLIP region (Ad-liCLIPGP: a double M to A point mutation - M91A M99A - designed to abolish li interaction with MHC class I molecules,) and the KEY motif (Ad-liKEYGP: a LRMK to AAAA quadriple point mutation which would destroy the li-Key segment). None of these mutations were key to the li mediated enhanced stimulation of CD8+ T cells. The only interesting data came when we combined N- and C-terminal truncations. Thus when we tested a 51-118 variant (Ad-li51-118GP), a pronounced reduction in CD8+ T cells stimulatory capacity was observed, but the mutant was still superior to the Ad-GP (figure 7).

### Example 3

In one embodiment of the invention, a non-human glycosyltransferase combined with glycosyl-binding proteins coupled to li is provided. li may be full length or a variant, wherein the variant may be a truncated version of li comprising residues number 50 to 215. This variant has full activity despite the lack of a transmembrane domain. Optionally, an adjuvant or one or more translocation domain may be further provided. In figure 15 is provided a schematic drawing of an embodiment wherein the Mannose receptor (a calcium-dependent lectin often targeted in vaccines) is coupled to a variant of invariant chain comprising residues 50 to 215 (li50-215), further coupled to an adenoviral fiber protein. The adenoviral fiber protein (Ad fiber) may stem from any serotype of adenovirus. The mannose receptor may be one or more domains from the Mannose receptor.

In one specific example, an Adenovirus expressing Egghead (a protein from Drosophila) in one reading frame, and expressing the Mannose receptor (or domains from the Mannose receptor) coupled to a variant of li having full activity without a transmembrane region such as the li50-215 variant further couplet to and adenoviral fiber protein in another reading frame is provided.

The glycosyltransferase such as Egghead and the glycosyl-binding proteins such as Mannose receptor may be expressed from different reading frames in the same Adenoviral vector, or the glycosyltransferase such as Egghead and the glycosyl-binding proteins such as Mannose receptor may be expressed from different Adenoviral vectors administered simultaneously.

Egghead couples Mannose on all glycosylated ER (endoplasmatic reticulum) proteins. The mannosylation of secreted proteins may thus cause the binding of mannosylated protein to the Mannose receptor-li-Ad fiber complex (as shown in figure 15). The Adenoviral fiber of the complex causes the secreted proteins linked to said complex to be taken up by other cells, activating these to become immune-stimulating and providing access of the complex to the cytosol where li may exert its effects.

This technology may be used to construct a vaccine that may be administered directly into for example cancers.

### Reference list

Andreasen, S. O., Christensen, J. E., Marker, O. & Thomsen, A. R. (2000). Role of CD40 ligand and CD28 in induction and maintenance of antiviral CD8+ effector T cell responses. J Immunol 164, 3689-3697.
Bartholdy, C., Stryhn, A., Hansen, N. J., Buus, S. & Thomsen, A. R. (2003). Incomplete effector/memory differentiation of antigen-primed CD8+ T cells in gene gun DNA-vaccinated mice. Eur J Immunol 33, 1941-1948.
Battegay,M., Cooper,S., Althage,A., Banziger,J., Hengartner,H., and Zinkernagel,R.M. (1991). Quantification of lymphocytic choriomeningitis virus with an immunological focus assay in 24- or 96-well plates. J. Virol.Methods 33:191-198.
Becker,T.C., Noel,R.J., Coats,W.S., Gomez-Foix,A.M., Alam,T., Gerard,R.D., and Newgard,C.B. (1994). Use of recombinant adenovirus for metabolic engineering of mammalian cells. Methods Cell Biol. 43 Pt A:161-189.
Christensen, J. P., Marker, O. & Thomsen, A. R. (1994). The role of CD4+ T cells in cell-mediated immunity to LCMV: studies in MHC class I and class II deficient mice. Scand J Immunol 40, 373-382.
Christensen, J. P., Kauffmann, S. O. & Thomsen, A. R. (2003). Deficient CD4+ T cell priming and regression of CD8+ T cell functionality in virus-infected mice lacking a normal B cell compartment. J Immunol 171, 4733-4741.
Diebold, S. S., M. Cotten, N. Koch, and M. Zenke. (2001). MHC class II presentation of endogenously expressed antigens by transfected dendritic cells. Gene Ther. 8:487-493.
Holmes JP, Benavides LC, Gates JD, Carmichael MG, Hueman MT, Mittendorf EA, Murray JL, Amin A, Craig D, von Hofe E, Ponniah S, Peoples GE. (2008). Results of the first phase I clinical trial of the novel II-key hybrid preventive HER-2/neu peptide (AE37) vaccine. J Clin Oncol. Jul 10;26(20):3426-33.
Holst, P. J., Sorensen, M. R., Mandrup Jensen, C. M., Orskov, C., Thomsen, A. R. & Christensen, J. P. (2008). MHC Class II-Associated Invariant Chain Linkage of Antigen Dramatically Improves Cell-Mediated Immunity Induced by Adenovirus Vaccines. J Immunol 180, 3339-3346.
Kallinteris NL, Lu X, Blackwell CE, von Hofe E, Humphreys RE, Xu M. (2006). li-Key/MHC class II epitope hybrids: a strategy that enhances MHC class II epitope loading to create more potent peptide vaccines. Expert Opin. Biol. Ther. 6(12):1311-1321.
Morris, C. R., A. J. Reber, J. L. Petersen, S. E. Vargas, and J. C. Solheim. (2004). Association of intracellular proteins with folded major histocompatibility complex class I molecules. Immunol.Res. 30:171-179.
Pieters, J. (1997). MHC class II restricted antigen presentation. Curr.Opin.Immunol. 9:89-96.
Sambrook et al., eds., Molecular Cloning: A Laboratory Manual, Cold Spring Harbor Laboratory, 2nd Edition, Cold Spring Harbor, N.Y., 1989.
Strumptner-Cuvelette, P., and P. Benaroch. (2002). Multiple roles of the invariant chain in MHC class II function. Biochem. Biophys. Acta., 1542:1-13.
WO 2007/062656
US 2008/0095798
WO 2003/089471
US 5,554,372
US 5,876,735
5,102,985

### Clauses to further illustrate the invention

Clause 1. A nucleic acid construct comprising sequences encoding
   a. at least one invariant chain or variant thereof operatively linked to
   b. at least one antigenic protein or peptide or an antigenic fragment of said protein or peptide,
   wherein said nucleic acid construct is used for priming of a cancer vaccine, and/or said invariant chain or variant thereof does not comprise the first 17 amino acids, and/or said invariant chain or variant thereof does not comprise the LRMK amino acid residues of the KEY region, and/or said invariant chain comprises a variant of the CLIP region.
Clause 2. The nucleic acid construct according to clause 1 for use as a primer for the stimulation of an immune response, thereby increasing the potency of a subsequently administered vaccine.
Clause 3. The nucleic acid construct according to clause 1, wherein at least one invariant chain or variant thereof is organism specific.
Clause 4. The nucleic acid construct according to any of the preceding clauses, wherein at least one invariant chain or variant thereof is of human origin.
Clause 5. The nucleic acid construct according to any of the preceding clauses, wherein at least one region, peptide or domain of the at least one invariant chain is added to, removed from or substitutes a region, peptide or domain of the at least one invariant chain.
Clause 6. The nucleic acid construct according to any of the preceding clauses, wherein one, two, three or four of the LRMK amino acid residues of the KEY region of the at least one invariant chain are substituted with other amino acid residues.
Clause 7. The nucleic acid construct according to any of the preceding clauses, wherein one, two, three or four of the LRMK amino acid residues of the KEY region of the at least one invariant chain are substituted with alanine.
Clause 8. The nucleic acid construct according to any of the preceding clauses, wherein one, two, three or four of the LRMK amino acid residues of the KEY region of the at least one invariant chain are deleted.
Clause 9. The nucleic acid construct according to any of the preceding clauses, wherein the methionine amino acid residues on positions 91 and 99 of the CLIP region of the at least one invariant chain are substituted with other amino acid residues.
Clause 10. The nucleic acid construct according to any of the preceding clauses, wherein the methionine amino acid residues on positions 91 and 99 of the CLIP region of the at least one invariant chain are substituted with alanine.
Clause 11. The nucleic acid construct according to any of the preceding clauses, wherein the first 17 amino acids of the at least one invariant chain are deleted (Δ17li).
Clause 12. The nucleic acid construct according to any of the preceding clauses, wherein the invariant chain comprises amino acid residues number 50 to 118 coupled to a trimerisation domain from another protein.
Clause 13. The nucleic acid construct according to any of the preceding clauses wherein the at least one invariant chain or fragments of same elicit an MHC-I and/or an MHC-II dependent immune response in a CD4⁺ T cell dependent and/or independent manner.
Clause 14. The nucleic acid construct according to any of the preceding clauses, wherein at least one antigenic protein or peptide or an antigenic fragment of said protein or peptide is selected from the group of: pathogenic organisms, cancer-specific polypeptides, and proteins or peptides associated with an abnormal physiological response.
Clause 15. The nucleic acid construct according to any of the preceding clauses, wherein the at least one antigenic protein or peptide or an antigenic fragment of said protein or peptide from a pathogenic organism is selected from the group of pathogens comprising: virus, micro organisms and parasites.
Clause 16. The nucleic acid construct according to any of the preceding clauses, wherein at least one antigenic protein or peptide or an antigenic fragment of said protein or peptide is from a mammalian organism, preferably a human pathogen.
Clause 17. The nucleic acid construct according to any of the preceding clauses, wherein at least one antigenic protein or peptide or an antigenic fragment of said protein or peptide is from a cancer-specific polypeptide.
Clause 18. The nucleic acid construct according to any of the preceding clauses, wherein at least one antigenic protein or peptide or an antigenic fragment of said protein or peptide is from a cancer-specific polypeptide selected from the group of: HPV derived viral oncogene E5, E6, E7 and L1; Survivin, Bcl-XL, MCL-1 and Rho-C.
Clause 19. The nucleic acid construct according to any of the preceding clauses, wherein at least one antigenic protein or peptide or an antigenic fragment of said protein or peptide is an antigenic peptide or protein at least 85% identical to any of the in clauses 14 to 18 described antigens.
Clause 20. The nucleic acid construct according to any of the preceding clauses, wherein the operative linker between the invariant chain or variant thereof and the antigenic protein or peptide or an antigenic fragment of said protein or peptide is selected from the group of: a direct link or a link mediated by a spacer region.
Clause 21. The nucleic acid construct according to clause 20, wherein the operative linker is a spacer region.
Clause 22. The nucleic acid construct according to clause 21 , wherein the spacer region encodes at least one helper epitope for class II MHC molecules.
Clause 23. The nucleic acid construct according to clause 21, wherein the spacer region encodes at least one protease cleavage site.
Clause 24. The nucleic acid construct according to any of the preceding clauses, wherein at least one invariant chain is operatively linked to at least two antigenic proteins or peptides or an antigenic fragment of said protein or peptide.
Clause 25. The nucleic acid construct according to any of the preceding clauses, wherein the at least one operatively linked invariant chain or variant thereof and at least one antigenic protein or peptide or an antigenic fragment of said protein or peptide encoding sequence is preceded by a promoter enabling expression of the construct.
Clause 26. The nucleic acid construct according to any of the preceding clauses, wherein the promoter is selected from the group of constitutive promoters, inducible promoters, organism specific promoters, tissue specific promoters and cell type specific promoters, CMV promoter, SV40 promoter, and RSV promoter.
Clause 27. The nucleic acid construct according to any of the preceding clauses, comprising genes related to antigen presentation and/or intercellular spreading.
Clause 28. The nucleic acid construct according to any of the preceding clauses, comprising genes encoding an adjuvant.
Clause 29. A delivery vehicle comprising the nucleic acid construct according to any of the preceding clauses.
Clause 30. The delivery vehicle according to clause 29, wherein the vehicle is selected from the group of: RNA based vehicles, DNA based vehicles/ vectors, lipid based vehicles, polymer based vehicles and virally derived DNA or RNA vehicles.
Clause 31. The delivery vehicle according to clause 30, wherein said delivery vehicle is a pegylated vector or vehicle.
Clause 32. The delivery vehicle according to clause 30, wherein said lipid based vehicle is a liposome.
Clause 33. The delivery vehicle according to clause 30, wherein said polymer based vehicle is formed of a cationic polymer DNA carrier selected from the group consisting of polyethylenimine (PEI), polyamidoamine and polypropylamine dendrimers, polyallylamine, cationic dextran, chitosan, cationic proteins (polylysine, protamine, and histones), cationic peptides, polypeptides, lipopolysaccharides and polysaccharides.
Clause 34. The delivery vehicle according to clause 33, wherein said delivery vehicles are biodegradable polymer microspheres.
Clause 35. The delivery vehicle according to clause 30, wherein said delivery vehicle comprises coating the nucleic acid construct onto colloidal gold particles.
Clause 36. A method for administering the delivery vehicle according to any of clauses 29 to 35, wherein said administration is selected from the group consisting of needle injection, gene gun, jet injection, electroporation, ultrasound, and hydrodynamic delivery.
Clause 37. A method for administering the nucleic acid construct according to any of clauses 29 to 35, wherein said administration is selected from the group consisting of needle injection, gene gun, jet injection, electroporation, ultrasound, and hydrodynamic delivery.
Clause 38. A cell comprising the nucleic acid construct as defined in any of the clauses 1 to 28.
Clause 39. A chimeric protein comprising at least one operatively linked invariant chain or variant thereof and at least one antigenic protein or peptide encoding sequence, as defined by any of the nucleic acid constructs in any of the clauses 1 to 28.
Clause 40. An antibody that can recognize the chimeric protein as defined in clause 39.
Clause 41. Use of the antibody according to clause 40, in an assay for detecting proteins to which the antibody binds.
Clause 42. A composition comprising a nucleic acid sequence encoding:
   a. at least one invariant chain or variant thereof operatively linked to
   b. at least one antigenic protein or peptide or an antigenic fragment of said protein or peptide.
Clause 43. A composition comprising a nucleic acid sequence encoding:
   a. at least one invariant chain or variant thereof operatively linked to
   b. at least one polypeptide
   for use as a medicament.
Clause 44. The composition according to any of the clauses 42 and 43, comprising a nucleic acid construct as defined in any of the clauses 1 to 28.
Clause 45. The composition according to any of the clauses 42 to 44, wherein the composition comprises at least two vectors.
Clause 46. A composition comprising at least one chimeric protein according to clause 39 as defined by any of the nucleic acid constructs in any of the clauses 1 to 28.
Clause 47. The composition according to any of the clauses 42 to 46, wherein the composition comprises an adjuvant.
Clause 48. A kit in parts comprising:
   a. a composition comprising a nucleotide construct according to any of the clauses 1 to 28
   b. a medical instrument or other means for administering the composition
   c. instructions on how to use the kit in parts.
Clause 49. The kit in parts according to clause 48, wherein said kit comprises a second active ingredient.
Clause 50. The kit in parts according to clause 49, wherein said second active ingredient is a suitable vaccine.
Clause 51. A method for increasing the potency of a vaccine comprising the steps of
   a. providing the nucleic acid construct according to any of clauses 1 -28, or the composition according to any of clauses 42 to 47,
   b. priming the immune system of a subject by administering the nucleic acid construct or composition of step a) thereby stimulating an immune response in said subject, and
   c. boosting the immune response of step b) by administering a suitable vaccine.
Clause 52. The method according to clause 51, wherein at least a portion of the nucleic acid construct used for priming an immune response and the vaccine used to boost said immune response is identical.
Clause 53. The method according to clause 52, wherein the identical portion is part of the antigenic peptide or protein.
Clause 54. The method according to clause 52, wherein the identical portion is an ubiquitious helper T cell epitope.
Clause 55. The method according to clause 51, wherein said immune response is MHC-I dependent.
Clause 56. The method according to clause 51, wherein said immune response is MHC-II dependent.
Clause 57. The method according to clause 51, wherein said immune response is MHC-I and MHC-II dependent
Clause 58. The method according to clause 51, wherein said immune response is T-cell dependent.
Clause 59. The method according to clause 51, wherein said immune response is CD4⁺ T-cell dependent.
Clause 60. The method according to clause 51, wherein said immune response is CD4⁺ T-cell independent.
Clause 61. The method according to clause 51, wherein said immune response is CD8⁺ T-cell dependent.
Clause 62. The method according to any of the clauses 51 to 61, comprising the steps of:
   a. providing at least one composition according to any of the clauses 42 to 47
   b. administering said at least one composition to a subject at least once for treatment or prophylaxis of an animal.
Clause 63. The method according to any of the clauses 51 to 62, wherein the composition is administered to the area most likely to be the receptacle of a given infection.
Clause 64. The method according to any of the clauses 51 to 63, wherein the subject is a human.
Clause 65. The method according to any of the clauses 51 to 63, wherein the subject is a mammal.
Clause 66. The method according to any of the clauses 51 to 63, wherein the subject is a ferret.
Clause 67. The method according to any of the clauses 51 to 63, wherein the subject is a fish.
Clause 68. The method according to any of the clauses 51 to 63, wherein the subject is a bird.

## Claims

1. A nucleic acid construct comprising sequences encoding
a. at least one invariant chain or variant thereof operatively linked to
b. at least one antigenic protein or peptide or an antigenic fragment of said protein or peptide,
wherein said nucleic acid construct is used for priming of a cancer vaccine, and/or said invariant chain or variant thereof does not comprise the first 17 amino acids, and/or said invariant chain or variant thereof does not comprise the LRMK amino acid residues of the KEY region, and/or said invariant chain comprises a variant of the CLIP region.

2. The nucleic acid construct according to claim 1, wherein the at least one invariant chain or variant thereof does not comprise the LRMK amino acid residues of the KEY region.

3. The nucleic acid construct according to either claim 1 or 2, wherein the at least one invariant chain or variant thereof is operatively linked at the terminal part after the last residue of the invariant chain or variant thereof to the at least one antigenic protein or peptide or an antigenic fragment of said protein or peptide.

4. The nucleic acid construct according to any one of claims 1 to 3, wherein at least one invariant chain or variant thereof is of human origin.

5. The nucleic acid construct according to any one of claims 1 to 4, wherein one, two, three or four of the LRMK amino acid residues of the KEY region of the at least one invariant chain are deleted.

6. The nucleic acid construct according to claim 5, wherein four of the LRMK amino acid residues of the KEY region of the at least one invariant chain are deleted.

7. The nucleic acid construct according to any one of claims 1 to 6, wherein at least one antigenic protein or peptide or an antigenic fragment of said protein or peptide is selected from the group of: pathogenic organisms, cancer-specific polypeptides, and proteins or peptides associated with an abnormal physiological response.

8. The nucleic acid construct according to any one of claims 1 to 7, wherein the at least one antigenic protein or peptide or an antigenic fragment of said protein or peptide from a pathogenic organism is selected from the group of pathogens comprising: virus, micro organisms and parasites.

9. The nucleic acid construct according to any one of claims 1 to 8, wherein at least one antigenic protein or peptide or an antigenic fragment of said protein or peptide is from a mammalian organism, preferably a human pathogen.

10. The nucleic acid construct according to any one of claims 1 to 9, wherein at least one antigenic protein or peptide or an antigenic fragment of said protein or peptide is from a cancer-specific polypeptide.

11. The nucleic acid construct according to any one of claims 1 to 10, wherein the operative linker between the invariant chain or variant thereof and the antigenic protein or peptide or an antigenic fragment of said protein or peptide is a direct link.

12. The nucleic acid construct according to any one of claims 1 to 10, wherein the operative linker between the invariant chain or variant thereof and the antigenic protein or peptide or an antigenic fragment of said protein or peptide is a spacer region.

13. A delivery vehicle comprising the nucleic acid construct according to any one of claims 1 to 12.

14. A composition comprising a nucleic acid construct according to any one of claims 1 to 12 for use as a medicament.

15. A method for increasing the potency of a vaccine comprising the steps of
a. providing the nucleic acid construct according to any of claims 1 -12,
b. priming the immune system of a subject by administering the nucleic acid construct or composition of step a) thereby stimulating an immune response in said subject, and
c. boosting the immune response of step b) by administering a suitable vaccine.
